# EUROPÄISCHE PATENTSCHRIFT

(11) **EP 3 459 531 B1**
(45) Veröffentlichungstag und Bekanntmachung des Hinweises auf die Patenterteilung: **28.10.2020**
(21) Anmeldenummer: 18213085.6
(22) Anmeldetag: 17.12.2018
(51) Int. Cl.: A61K 9/20, A61K 9/24, A61K 31/4415, A61K 31/51, A61K 31/525, A61K 31/714, A61K 33/06, A61P 3/12

(54) **VERFAHREN ZUR HERSTELLUNG EINER SCHICHTTABLETTE**
METHOD FOR PRODUCING A LAYERED TABLET
PROCÉDÉ DE FABRICATION D'UN COMPRIMÉ STRATIFIÉ

(43) Veröffentlichungstag der Anmeldung: 27.03.2019
(73) Patentinhaber: Protina Pharmazeutische Gesellschaft mbH, 85737 Ismaning (DE)
(72) Erfinder: Flamm, Stefan, 82110 Germering (DE); Barthel, Wolfgang, 85399 Halbergmoos (DE); Schumi, Emanuel, 81369 München (DE); WERNER, Tanja, 81825 München (DE)
(74) Vertreter: Eisenführ Speiser

(56) Entgegenhaltungen:
- EP-A1- 3 357 492
- WO-A1-99/66943
- ASKARI GHOLAMREZA ET AL: "The effects of folic acid and pyridoxine supplementation on characteristics of migraine attacks in migraine patients with aura: A double-blind, randomized placebo-controlled, clinical trial", NUTRITION, ELSEVIER INC, US, Bd. 38, 2. Februar 2017 (2017-02-02), Seiten 74-79, XP085026997, ISSN: 0899-9007, DOI: 10.1016/J.NUT.2017.01.007

## Beschreibung

Die vorliegende Erfindung betrifft primär ein Verfahren zur Herstellung einer Schichttablette umfassend oder bestehend aus einer ersten, Mg²⁺-Ionen freisetzenden, Schicht und einer zweiten, Mg²⁺-Ionen freisetzenden, Schicht wie hierin beschrieben.

Zudem betrifft die vorliegende Erfindung eine Schichttablette erhalten durch oder erhältlich gemäß einem Verfahren wie hierin beschrieben, eine Schichttablette umfassend oder bestehend aus einer ersten, Mg²⁺-Ionen freisetzenden Schicht und einer zweiten, Mg²⁺-Ionen freisetzenden Schicht, die die hierin beschriebenen Bestandteile enthält oder aus ihnen besteht, und eine Schichttablette wie hierin beschrieben zur Anwendung als Medikament.

Weitere Aspekte der vorliegenden Erfindung sowie besonders geeignete Ausgestaltungen ergeben sich aus der folgenden Beschreibung, den Beispielen sowie den beigefügten Patentansprüchen.

Als "Schichttabletten" werden auf dem Gebiet der Pharmazeutischen Technologie Tabletten bezeichnet, die aus verschiedenen, fest aneinanderhaftenden, parallelen bzw. konzentrisch gewölbten Schichten aus komprimierten Pulver- oder Granulatpartikeln bestehen (vgl. Hunnius Pharmazeutisches Wörterbuch, 6.Auflage, Berlin 1986). Konventionelle Schichttabletten werden üblicherweise in einem Arbeitsgang und auf einer Tablettenpresse durch mehrmals aufeinanderfolgendes Pressen unterschiedlicher Teilchenarten gefertigt, wobei - bedingt durch die Verwendung desselben Presswerkzeugs bei jeder Pressung - die Schichten eine charakteristische Form erhalten. Die Verwendung von biplanen Stempelwerkzeugen führt zu Schichttabletten mit durchweg parallelen Schichten, wenn man von den Randbezirken im Bereich der optionalen Facette absieht. Bei der Verwendung von gewölbten Stempeln wird die untere, zuerst gepresste Schicht regelmäßig bikonvex, während die obere bzw. zweite Schicht eine gleichmäßige Dicke besitzt und parallel zur gewölbten oberen Begrenzungsfläche der zuerst gepressten Schicht verläuft. Synonym zu verwenden mit dem Begriff der Schichttablette ist der Begriff der Mehrschichttablette. Eine Schichttablette oder Mehrschichttablette ist vorzugsweise aus zwei, drei oder mehreren Schichten aufgebaut.

Eine Schichttablette im Sinne der vorliegenden Erfindung besteht aus mindestens einem räumlich zusammenhängenden Teil, der aus mindestens einer ersten Mischung (wie hierin beschrieben) besteht und aus mindestens einem weiteren, räumlich zusammenhängenden Teil, der aus mindestens einer zweiten Mischung (wie hierin beschrieben) besteht. Vorzugsweise ist die erfindungsgemäße Schichttablette wie hierin beschrieben eine Zweischichttablette, d.h. eine Schichttablette, die aus zwei Schichten aufgebaut ist.

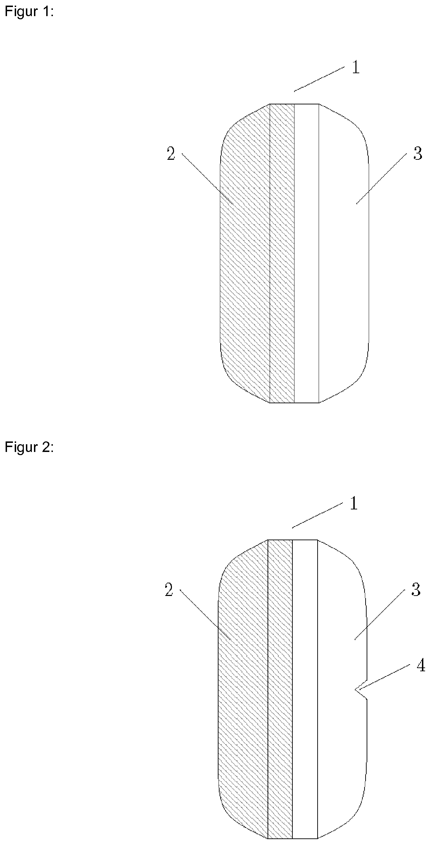

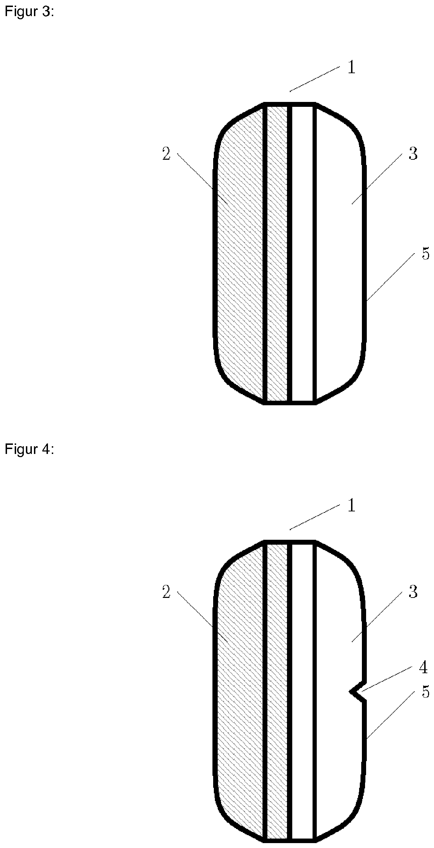

Der Begriff Schichttablette im Sinne der vorliegenden Erfindung bezeichnet sowohl eine Schichttablette ohne als auch mit dem Vorhandensein eines Films zum Zwecke der Beschichtung. Zur Verdeutlichung sei an dieser Stelle auf die hierin gezeigten Figuren 1 bis 4 verwiesen.

Schichttabletten und ein Verfahren zu ihrer Herstellung wurden bereits 1917 beschrieben (US-Patent 1,248,571). Sie werden in der Pharmazie bzw. Pharmazeutischen Technologie aus verschiedenen Überlegungen heraus eingesetzt. Zum einen bieten sie die Möglichkeit, inkompatible Wirkstoffe räumlich voneinander getrennt in einer Tablette zu kombinieren. Zum anderen erlauben sie die Kombination mehrerer Rezepturen mit unterschiedlichen Freisetzungseigenschaften. So lässt sich zum Beispiel die eine Schicht zur Abgabe einer Initialdosis des Wirkstoffes als schnellzerfallende, schnellfreisetzende Formulierung einsetzen, während die zweite Schicht eine Erhaltungsdosis in retardierter Form enthält. In diesem Zusammenhang sei auch die europäische Patentanmeldung EP 3357492 A1 genannt, welche eine Schichttablette zur Gabe von Magnesium offenbart.

Eine Schichttablette im Sinne der vorliegenden Erfindung sorgt für eine verzögerte, möglichst gleichmäßige (Wirkstoff-)Abgabe aus einem (Wirkstoff-)Vorrat unter Vermeidung von Konzentrationsspitzen über eine bestimmte Zeit. Dadurch können die totalen (Wirkstoff-)Mengen oft reduziert werden, gegebenenfalls (Arzneimittel-)Nebenwirkungen verringert werden und eine mehrmalige Dosierung durch eine einmalige Gabe ersetzt werden. Die Anforderungen an eine ideale Schichttablette, die unter realen Bedingungen in der Regel nicht vollständig erreicht werden können, lassen sich also im Wesentlichen folgendermaßen zusammenfassen:
- Von der Zubereitung bzw. Schichttablette wird nach der Applikation ein rasches Erreichen der optimalen Werte, d.h. der gewünschten Konzentration des (Wirk)stoffs verlangt.
- Es ist ein konstantes Niveau zu sichern.
- Über die gewünschte Zeitdauer muss eine gleichmäßige Wirkung erhalten bleiben.
- Durch die Vermeidung von Konzentrationsspitzen, d.h. Verhinderung des Vordringens der (Wirkstoff-)Konzentration in den toxischen Bereich, sind Intensität und Häufigkeit unerwünschter Nebenwirkungen zu reduzieren.

Im Zusammenhang mit einer verzögerten, möglichst gleichmäßigen (Wirkstoff-)Abgabe aus einem (Wirkstoff-)Vorrat (wie hierin beschrieben) werden vorzugsweise die beiden folgenden Typen von Depotarzneiformen eingesetzt:
- Sustained-release-Typ (Synonyme: gleichmäßig anhaltende Wirkstofffreigabe, sustained action): Aus einer Darreichungsform wird der Wirkstoff durch eine Initialdosis dem Körper in einer Konzentration zugänglich gemacht, die die gewünschte (pharmakodynamische) Wirkung ergibt und die eine Erhaltung dieser optimalen Konzentration für eine gewisse Zeit über die Wirkungszeit einer Einzeldosis hinaus garantiert.
- Prolonged-release-Typ (Synonyme: verlängerte (protrahierte) Wirkstofffreigabe, prolonged action): Aus einer Darreichungsform wird der Wirkstoff durch eine Initialdosis dem Körper in einer Menge zugänglich gemacht, die genügend, aber nicht unerwünscht hoch ist und den gewünschten Effekt ausübt. Zudem soll diese Darreichungsform den Wirkstoff kontinuierlich so freigeben, dass eine messbare Wirkungsverlängerung gegenüber einer normalen Einzeldosis resultiert.

"Nahrungsergänzungsmittel" im Sinne der vorliegenden Erfindung (wie unten beschrieben) sind Erzeugnisse zur erhöhten Versorgung des menschlichen Stoffwechsels mit bestimmten Nähr-, Mineral- und/oder Wirkstoffen im Grenzbereich zwischen Arzneimitteln und Lebensmitteln.

Ein Mangel an Magnesium kann unter anderem an der Entwicklung von Beschwerden wie Depressionen, Menstruationsbeschwerden, Krämpfen, Muskelschwäche, Migräne, Schlafstörungen, Nervosität, Magen-Darm-Problemen, Herz-Kreislauf-Erkrankungen, Bluthochdruck, Reizbarkeit, chronischen Schmerzzuständen, Vitamin-D-Mangel (Magnesium ist an der Aktivierung des Vitamin D beteiligt), Diabetes oder Schwangerschaftsbeschwerden beteiligt sein. Ein erhöhter Bedarf an Magnesium kann sich ebenso ergeben bei Einnahme von Medikamenten (manche Blutdrucksenker, Cortison, die Antibabypille, Immunsuppressiva, Methotrexat (z. B. gegen Rheuma), etc.), in der Schwangerschaft und der Stillzeit, beim (Leistungs-)Sport, im Alter, in Stresssituationen (Stresshormone sorgen für eine erhöhte Magnesiumausscheidung mit dem Urin), bei Nierenerkrankungen, bei Magersucht, bei chronisch-entzündlichen Darmerkrankungen, bei einer Aluminiumbelastung (Aluminium verdrängt Magnesium), bei Vitamin B1- und/oder B6-Mangel und während der Rekonvaleszenz.

"Magnesium" bzw. ein "Mangel an Magnesium" ist im Sinne der vorliegenden Erfindung vorzugsweise zu verstehen als physiologisch resorbierbares Magnesium, d.h. Mg²⁺-Ionen bzw. deren Mangel. Magnesium übt im Körper vielfältige Funktionen aus. Daher führt eine unzureichende Versorgung zu Mangelsymptomen. Der Magnesiumhaushalt des Körpers wird durch die intestinale Resorption, die biliäre und renale Ausscheidung sowie die Speicherung im Skelett reguliert. Die Resorption von Magnesium erfolgt über den kompletten Darm, vorzugsweise im oberen Dünndarm, teils parazellulär durch passive Diffusion. Die Resorptionsrate aus der Nahrung liegt regelmäßig bei 35 bis 55%. (vgl. Klaus Eder, "Magnesium und Mg-Verbindungen in Supplementen", Deutsche Apothekerzeitung 26/2008).

Trotz einer Vielzahl bekannter Magnesium-haltiger Nahrungsergänzungsmittel besteht unter anderem in der Nahrungsergänzungsmittelindustrie auch weiterhin ein generelles Bedürfnis nach neuen Nahrungsergänzungsmitteln, die über ihre primäre Funktion des Versorgens eines Organismus mit Magnesium hinaus vorzugsweise zusätzliche positive Eigenschaften, wie Verringerung von Nebenwirkungen und/oder gezielte Wirkstofffreisetzung mit sich bringen.

Die primäre technische Aufgabe der vorliegenden Erfindung war es, ein neues Verfahren zur Herstellung einer Schichttablette bereitzustellen, vorzugsweise einer solchen, die sich gegenüber dem Stand der Technik durch zusätzliche verbesserte Eigenschaften im Sinne eines definierten vorteilhaften Freisetzungsprofils des enthaltenen Magnesiums auszeichnet. Zudem sollten entsprechend hergestellte neue, vorteilhafte Schichttabletten und neue Anwendungen davon bereitgestellt werden.

Weitere Aufgabenstellungen, die der vorliegenden Erfindung zu Grunde liegen, ergeben sich aus den nachfolgenden Ausführungen und den beigefügten Patentansprüchen.

Die vorangehend genannte primäre Aufgabe der vorliegenden Erfindung wird demnach erfindungsgemäß gelöst durch ein Verfahren zur Herstellung einer Schichttablette umfassend oder bestehend aus einer ersten, Mg²⁺-Ionen freisetzenden, Schicht und einer zweiten, Mg²⁺-Ionen freisetzenden, Schicht umfassend oder bestehend aus den Schritten
I) Bereitstellen
   A) einer ersten Mischung zur Herstellung der ersten Schicht, wobei die erste Mischung die folgenden Bestandteile enthält oder aus den folgenden Bestandteilen besteht:
      A-i) einem oder mehreren Magnesium-Salzen ausgewählt aus der Gruppe, bestehend aus Magnesiumoxid, Magnesiumhydroxid, Magnesiumcarbonat, Trimagnesiumdicitrat, Magnesiumgluconat, Magnesiummalat, Magnesiumglycinat, Magnesiumorotat, Magnesiumacetat, Magnesiumascorbat, Magnesiumformiat, Magnesiumfumarat, Magnesiumglutamat, Magnesiumglycerophosphat, Magnesiumlactat, Magnesiumnitrat, Magnesiumoxalat, Magnesiumphosphat, Magnesiumpidolat, Magnesiumpropionat, Magnesiumtartrat, Magnesiumaspartat, Magnesiumchlorid und Magnesiumsulfat,
      A-ii) einem oder mehreren Vitaminen, vorzugsweise B-Vitaminen,
      A-iii) einem oder mehreren Sprengmitteln,
      A-iv) einem oder mehreren Trägerstoffen, und optional
      A-v) einem oder mehreren Pigmenten oder Farbstoffen, und optional
      A-vi) einem oder mehreren (weiteren) Hilfsstoffen,
      und
   B) einer zweiten Mischung zur Herstellung der zweiten Schicht, wobei die zweite Mischung die folgenden Bestandteile enthält oder aus den folgenden Bestandteilen besteht:
      B-i) mindestens einer ersten Komponente, umfassend oder bestehend aus:
         B-i-a) einem oder mehreren Magnesium-Salzen ausgewählt aus der Gruppe, bestehend aus Magnesiumoxid, Magnesiumhydroxid, Magnesiumcarbonat, Trimagnesiumdicitrat, Magnesiumgluconat, Magnesiummalat, Magnesiumglycinat, Magnesiumorotat, Magnesiumacetat, Magnesiumascorbat, Magnesiumformiat, Magnesiumfumarat, Magnesiumglutamat, Magnesiumglycerophosphat, Magnesiumlactat, Magnesiumnitrat, Magnesiumoxalat, Magnesiumphosphat, Magnesiumpidolat, Magnesiumpropionat, Magnesiumtartrat, Magnesiumaspartat, Magnesiumchlorid und Magnesiumsulfat,
         B-i-b) einem oder mehreren Tablettierhilfsmitteln, und
         B-i-c) einem oder mehreren Trägerstoffen zur Feuchtgranulierung
         und
      B-ii) mindestens einer zweiten Komponente, umfassend oder bestehend aus:
         B-ii-a) einem oder mehreren Vitaminen, vorzugsweise B-Vitaminen, und optional
         B-ii-b) einem oder mehreren (weiteren) Hilfsstoffen,
   wobei die bzw. mindestens eine Komponente der Zusammensetzung B-i) durch ein Verfahren zur Feuchtgranulierung, vorzugsweise der Wirbelschichtgranulation, hergestellt ist,
   wobei B-i) und B-ii) miteinander gemischt sind, und
   wobei die Mischungen A) und B) unabhängig voneinander jeweils als Granulat oder Pulver, vorzugsweise als Feuchtgranulat, bereitgestellt werden,
II) Vorpressen der Mischung A) oder der Mischung B), um einen Schichttabletten-Vorpressling zu erhalten,
III) Hinzufügen der nicht vorgepressten Mischung A) oder B),
IV) gemeinsames Verpressen der Mischungen A) und B) zu einer Schichttablette, und optional
V) Befilmen der Schichttablette, um einen Film C) auf der Oberfläche der Schichttablette zu erhalten,
dadurch gekennzeichnet, dass Mg²⁺-Ionen aus der ersten Schicht gegenüber Mg²⁺-Ionen aus der zweiten Schicht bei Körpertemperatur unter atmosphärischem Druck in Wasser schneller freigesetzt werden.

"Bereitstellen" im Sinne der vorliegenden Erfindung bedeutet das Vorlegen der jeweiligen Mischungen bzw. Bestandteile in einem geeigneten Behältnis oder einer Mehrzahl solcher Behältnisse, bevorzugt unter kontrollierten Bedingungen, umfassend Druck und Temperatur, bevorzugt mit dem Ziel, die nachfolgenden Verfahrensschritte zur Herstellung erfindungsgemäßer Schichttabletten zu ermöglichen, vorzugsweise zu erleichtern. Das Bereitstellen erfolgt vorzugsweise getrennt, wobei das "Getrennte Bereitstellen" die räumliche und/oder zeitliche Trennung des Bereitstellens der Mischungen, die zur Durchführung des erfindungsgemäßen Verfahrens erforderlich sind, beschreibt. Beispielsweise bedeutet räumliche Trennung, das eine erste Mischung in einem ersten Behältnis und eine zweite Mischung in einem zweiten Behältnis bereitgestellt wird. Zeitliche Trennung des Bereitstellens bedeutet beispielsweise, dass eine erste Mischung zeitlich vor einer zweiten, d.h. nachfolgend bereitgestellten, Mischung bereitgestellt wird, wobei die zuerst bereitgestellte Mischung vor der Bereitstellung der nachfolgenden Mischung optional für eine bestimmte Zeit gelagert bzw. aufbewahrt wird, vorzugsweise unter dem Fachmann bekannten Standardbedingungen oder Bedingungen, die gezielt eine Veränderung der bereitgestellten Mischung herbeiführt oder eine derartige Änderung gezielt unterbindet. Die räumliche und/oder zeitliche Trennung des Bereitstellens sind im Sinne der vorliegenden Erfindung unabhängig voneinander.

Ein "Wirkstoff" im Sinne der vorliegenden Erfindung ist insbesondere ein Stoff, der befähigt ist, Magnesium bzw. Mg²⁺-Ionen in den erfindungsgemäßen Erzeugnissen freizusetzen und vorzugsweise damit einer der Grundlage für die vorausgehend beschriebene Resorption von Magnesium bzw. Mg²⁺-Ionen im Körper, die Anwesenheit besagter Spezies, zu gewährleisten.

Eine "erste Mischung" im Sinne der vorliegenden Erfindung bezeichnet eine Mischung, bestehend aus oder enthaltend einen oder mehrere Bestandteil(e) ausgewählt aus der Gruppe, bestehend aus A-i) Magnesium-Salzen oder Mg²⁺-freisetzenden Verbindungen, A-ii) Vitaminen, A-iii) Sprengmitteln, A-iv) Trägerstoffen, und optional A-v) Pigmenten und/oder Farbstoffen, und optional A-vi) sonstigen Hilfsstoffen. Kennzeichnend für eine erste Mischung bzw. eine daraus erhaltene bzw. erhältliche erste Schicht als Bestandteil einer erfindungsgemäßen Schichttablette ist die beschleunigte Freisetzung von Mg²⁺-Ionen im Vergleich zur Freisetzung von Mg²⁺-Ionen aus einer entsprechend zusammengesetzten zweiten Mischung bzw. zweiten Schicht, vorzugsweise bei Körpertemperatur unter atmosphärischem Druck in Wasser (vgl. hierzu die Ausführungen im Beispielteil unten).

Hinsichtlich der Bestandteile einer bzw. der ersten Mischung zur erfindungsgemäßen Herstellung einer ersten Schicht gilt, dass jeder der Bestandteile jeweils einer oder mehrerer Kategorien ausgewählt aus der Gruppe, bestehend aus A-i) Magnesium-Salzen oder Mg²⁺-freisetzenden Verbindungen, A-ii) Vitaminen, A-iii) Sprengmitteln, A-iv) Trägerstoffen, und optional A-v) Pigmenten und/oder Farbstoffen, und optional A-vi) sonstigen Hilfsstoffen zugeordnet bzw. zugerechnet werden kann (z.B. Magnesiumcarbonat als A-i) MagnesiumSalz oder Mg²⁺-freisetzende Verbindung und als A-iii) Sprengmittel).

Eine "zweite Mischung" im Sinne der vorliegenden Erfindung bezeichnet eine Mischung, bestehend aus oder enthaltend einen oder mehrere Bestandteil(e) ausgewählt aus der Gruppe, bestehend aus mindestens einer ersten Komponente B-i), d.h. mindestens einer ersten Komponente, umfassend oder bestehend aus: B-i-a) einem oder mehreren Magnesium-Salzen ausgewählt aus der Gruppe, bestehend aus Magnesiumoxid, Magnesiumhydroxid, Magnesiumcarbonat, Trimagnesiumdicitrat, Magnesiumgluconat, Magnesiummalat, Magnesiumglycinat, Magnesiumorotat, Magnesiumacetat, Magnesiumascorbat, Magnesiumformiat, Magnesiumfumarat, Magnesiumglutamat, Magnesiumglycerophosphat, Magnesiumlactat, Magnesiumnitrat, Magnesiumoxalat, Magnesiumphosphat, Magnesiumpidolat, Magnesiumpropionat, Magnesiumtartrat, Magnesiumaspartat, Magnesiumchlorid und Magnesiumsulfat, B-i-b) einem oder mehreren Tablettierhilfsmitteln, und B-i-c) einem oder mehreren Trägerstoffen zur Feuchtgranulierung, und mindestens einer zweiten Komponente B-ii), d.h. mindestens einer zweiten Komponente, umfassend oder bestehend aus: B-ii-a) einem oder mehreren Vitaminen, vorzugsweise B-Vitaminen, und optional B-ii-b) einem oder mehreren Hilfsstoffen, vorzugsweise weiteren Hilfsstoffen. Kennzeichnend für eine zweite Mischung bzw. eine daraus erhaltene bzw. erhältliche zweite Schicht als Bestandteil einer erfindungsgemäßen Schichttablette ist die verlangsamte Freisetzung von Mg²⁺-Ionen im Vergleich zur Freisetzung von Mg²⁺-Ionen aus einer entsprechend zusammengesetzten ersten Mischung bzw. ersten Schicht als Bestandteil einer erfindungsgemäßen Schichttablette wie hierin beschrieben, vorzugsweise bei Körpertemperatur unter atmosphärischem Druck in Wasser (vgl. hierzu die Ausführungen im Beispielteil unten).

Ein "Vitamin B" im Sinne der vorliegenden Erfindung ist vorzugsweise zu verstehen als ein Stoff ausgewählt aus der Gruppe der Substanzen bzw. Vorstufen von Coenzymen, die dem Fachmann auf dem Gebiet der Pharmazeutischen Technologie und unter Heranziehung des allgemeinen Fachwissens auf dem Gebiet ohne Weiteres bekannt sind. Zum Vitamin B-Komplex zählen Vitamin B1 (Thiamin), Vitamin B2 (Riboflavin, Laktoflavin), Vitamin B3 (Niacin, Nicotinsäure und ihr Amid, PP-Faktor), Vitamin B5 (Panthothensäure), Vitamin B6 (Sammelbezeichnung für Pyridoxin, Pyridoxamin und Pyridoxal), Vitamin B7 (Biotin; vormals als Vitamin H bezeichnet), Vitamin B9 (Folsäure-Gruppe, Folsäure, Folat, Pteroylglutaminsäure; vormals als Vitamin B11 oder Vitamin M bezeichnet), Vitamin B12 (Cobalamine). Daneben können noch p-Aminobenzoesäure, Inosit und Cholin (Vitamin B4) zu den B-Vitaminen gezählt werden. Die Zusammenfassung unter den Begriff des B-Komplexes lässt sich auch aufgrund ihrer Wasserlöslichkeit rechtfertigen und ist insbesondere durch die historische Entwicklung der Forschung auf dem Gebiet der Vitamine bedingt (J. Schormüller, Lehrbuch der Lebensmittelchemie, 2. Auflage, Springer-Verlag Berlin, Heidelberg, New York, 1974).

Ein "Sprengmittel" im Sinne der vorliegenden Erfindung ist vorzugsweise ein Stoff bzw. eine Mischung, der bzw. die dazu beiträgt, dass eine Tablette oder sonstige Darreichungsform in Wasser und/oder Magensaft, vorzugsweise möglichst schnell und/oder gleichmäßig, zerfällt. Sie werden auch als Zerfallsmittel bezeichnet. Die Wirkung von Sprengmitteln wird unter anderem beeinflusst von Art und Menge des verarbeiteten Wirkstoffs bzw. der verarbeiteten Wirkstoffe, Größe, Form und Feuchtigkeitsgehalt des verarbeiteten Granulats, der aufgewendeten Presskraft zur Herstellung der Tablette oder sonstigen Darreichungsform bzw. deren Härte und der Anwesenheit weiterer Hilfsstoffe, insbesondere Granuliermittel und Gleitmittel, in der Tablette oder sonstigen Darreichungsform. Sprengmittel werden in der Regel über ihre Zugehörigkeit zu einer oder mehrerer der im Folgenden kurz beschriebenen drei Gruppen klassifiziert:
- Substanzen, die die Kapillarität erhöhen, Feuchtigkeit absorbieren und quellen (Stärke, insbesondere Maisstärke und Kartoffelstärke, Alginsäure, sowie deren Salze und Derivate, insbesondere Calciumalginate und Natriumalginate, Natriumcarboxymethylcellulose, Polyacrylsäure, vernetztes Polyvinylpyrrolidon, vernetzte Natriumcarboxymethylcellulose, quervernetzte Natriumcarboxymethylstärke, niedrigsubstituierte Natriumcarboxymethylcellulose),
- Verbindungen, die bei Einwirkung von Feuchtigkeit unter Gasentwicklung aufbrausen (Natriumhydrogencarbonat, Magnesiumperoxid), und
- Substanzen, die die Benetzbarkeit der Tabletten erhöhen bzw. Hydrophilisierungsmittel (Natriumlaurylsulfat, nichtionogne Tenside Tween® 20 (Polyoxyethylen(20)-sorbitan-monolaurat), Tween® 60 (Polyoxyethylen(20)-sorbitan-monostearat), Tween® 80 (Polyoxyethylen(20)-sorbitan-monooleat), hochdisperses SiO₂, Cellulose) (R. Voigt, Pharmazeutische Technologie, 10. Auflage, Deutscher Apotheker Verband Stuttgart).

Ein "Trägerstoff" im Sinne der vorliegenden Erfindung ist ein Stoff bzw. Stoffgemisch ausgewählt aus der Gruppe, bestehend aus quervernetzter Natrium Carboxymethylcellulose, amorpher hochdisperser Kieselsäure, Stärke, Laktose, Maltose, Dextrose, Saccharose und Sorbit. Ihm kommt regelmäßig die Aufgabe zu, dabei zu helfen, andere Stoffe wie beispielsweise Farbstoffe, Aromen oder Vitamine in einem Lebensmittel kontrolliert zu verteilen. Trägerstoffe selbst haben vorzugsweise keine technologische Wirkung innerhalb des herzustellenden Erzeugnisses, erleichtern aber den Einsatz und die Handhabung von Lebensmittelzusatzstoffen und anderen, für die industrielle Lebensmittelherstellung verwendete Substanzen. Sie werden regelmäßig auch als technische Hilfsstoffe bezeichnet.

"Pigmente und Farbstoffe" im Sinne der vorliegenden Erfindung sind dem Fachmann auf dem Gebiet der Pharmazeutischen Technologie bekannt und können unter Heranziehung von allgemeinem Fachwissen auf dem Gebiet ohne Weiteres ausgewählt und hinsichtlich der maßgeblichen Kriterien, insbesondere im Lichte der aktuellen Zulassungen bzw. Zulassungsvoraussetzungen im Gebiet der Lebensmittelindustrie und/oder Nahrungsergänzungsmittelindustrie und/oder Pharmazie, evaluiert und verwendet werden.

Ein "Hilfsstoff" im Sinne der vorliegenden Erfindung ist vorzugsweise ein insbesondere von dem Wirkstoff oder den Wirkstoffen verschiedener Stoff in einer Tablette oder sonstigen Darreichungsform, dem mindestens eine Funktion bei der Vorbereitung zur Herstellung, der Herstellung, Lagerung oder sonstigen Funktion im Lebenszyklus der Tablette zukommt. Bevorzugt sind Hilfsmittel indifferent, im gewünschten Milieu inert bzw. pharmakologisch und toxikologisch inert, geruchslos, geschmacklos, und (möglichst) farblos. Hilfsstoffe im Sinne der vorliegenden Erfindung umfassen regelmäßig Sprengmittel, Trägerstoffe, Pigmente, Farbstoffe und Tablettierhilfsmittel. Ein Hilfsstoff ist vorzugsweise ausgewählt aus der Gruppe, bestehend aus Füllmitteln, Bindemitteln, Gleitmitteln, Zerfallsmitteln, Feuchthaltemitteln, Adsorptionsmitteln und Gegensprengmitteln. Im Sinne der vorliegenden Erfindung ist der eine gegebenenfalls vorhandene (weitere) Hilfsstoffe A-vi) und/oder B-ii-c) bzw. sind die gegebenenfalls vorhandenen mehreren (weiteren) Hilfsstoffe A-vi) und/oder B-ii-c) vorzugsweise verschieden von dem hierin beschriebenen einen Sprengmittel oder mehreren Sprengmitteln A-iii), einen Trägerstoff oder mehreren Trägerstoffen A-iv), einen Pigment und/oder Farbstoff oder mehreren Pigmenten und/oder Farbstoffen A-v), und einen Tablettierhilfsmittel oder mehreren Tablettierhilfsmitteln B-i-b). Dabei ist zu beachten, dass eine strenge Klassifizierung der Hilfsstoffe in Gruppen bzw. Kategorien nicht ohne weiteres möglich ist, da einzelne der Substanzen mehrere Funktionen haben können. Daher werden im Sinne der vorliegenden Erfindung der bzw. die gegebenenfalls vorhandene(nen) (weitere/n) Hilfsstoffe A-vi) und/oder B-ii-c) diejenigen Hilfsstoffe bezeichnet, deren hauptsächliche, charakteristische oder wesentliche Funktion der, der hierin beschriebenen Sprengmittel A-iii), Trägerstoffe A-iv), Pigmente und/oder Farbstoffe A-v), und Tablettierhilfsmittel B-i-b) entspricht. Dies schließt im Sinne dervorliegenden Erfindung jedoch nicht aus, dass die gegebenenfalls vorhandene(nen) (weitere/n) Hilfsstoffe A-vi) und/oder B-ii-c) untergeordnet, nebensächlich oder unwesentliche eine oder mehrere Funktionen der hierin beschriebenen Sprengmittel A-iii), Trägerstoffe A-iv), Pigmente und/oder Farbstoffe A-v), und Tablettierhilfsmittel B-i-b) erfüllen, vorzugsweise zu einem vernachlässigbaren Grad erfüllen. Hilfsstoffe zeichnen sich weiterhin vorzugsweise aus durch Erfüllen einer oder mehrerer Funktionen, ausgewählt aus der Gruppe aus Formgebung, Ermöglichen der Herstellbarkeit des Produkts, Steuerung der gezielten örtlichen und/oder zeitlichen Freisetzung der aktiven Verbindung und Stabilitätsverbesserung aus.

Exemplarisch können Hilfsstoffe ausgewählt sein aus der Gruppe bestehend aus:
- Füllstoffe oder Grundlage (z.B. Lactose, Cellulose, Stärken, Saccharose, Paraffin, Hartfett, Polyethylenglykole, Polyethylenoxide),
- Lösungsmittel und Befeuchtungsmittel (z.B. Wasser, Ethanol, Isopropanol),
- Emulgatoren (z.B. Cetylstearylalkohol, Gylcerolmonostearat, Lecithin, Fettsäureester des Sorbitans, des Polyoxyethylensorbitans (Polysorbate), des Polyoxyethylens, Polyoxyethylenfettalkoholether),
- Lösungsvermittler oder Netzmittel (z.B. Polyethylenglykole, Polyethylenoxide, Polysorbate),
- Puffer (z.B. Natriumdihydrogenphosphat, Natriumhydrogencarbonat, Calciumhydrogenphosphat, Trometamol),
- Verdickungs- und Bindemittel (z.B. Stärken, Guaran, Xanthan, Alginat, Carrageen, Pektin, Traganth, Polyacrylsäuren, Polyvinylpyrrolidon, hochdisperses Siliciumdioxid, substituierte Celluloseether, wie beispielsweise Methylcellulose, Ethylcellulose, Hydroxypropylcellulose, Hydroxypropylmethylcellulose, Carboxymethylcellulose),
- Trockenbindemittel (z.B. Cellulosen, Stärke, Hexite, Dextrose, Lactose, anorganische Calciumverbindungen, synthetische Polymere),
- Bindemittel oder Granulattablettierung (z.B. Gelatine, Stärkekleister, Gummi Arabicum, Tragant, synthetische Polymere),
- Umhüllungsmittel (z.B. Saccharose, vorzugsweise aus Zuckerdragierung, Gelatine, Gelatinepolysuccinat, Polyacrylate, Ethylcellulose, Methylcellulose),
- Gleit- und Schmiermittel oder Formentrennmittel (z.B. Polyethylenglykole, Polyethylenoxide, Talkum, Magnesiumstearat, Natriumlaurylsulfat),
- Filmbildner (z.B. Gelatine, Hydroxypropylmethylcellulose, Stärke, Carrageen, Polyvinylalkohol-Coplymere),
- Weichmacher (z.B. Glycerol, Sorbitol),
- Oberflächenaktive Substanzen (z.B. Natriumlaurylsulfat),
- Konservierungsmittel (z.B. p-Hydroxybenzoesäureester),
- Fließregulierungsmittel (z.B. Hochdisperses Siliciumdioxid, Aerosil, Metallstearate, Fettsäuren, Wachse, Fettalkohole),
- Antioxidantien (z.B. Butylhydroxytoluol, all-rac-α-Tocopherol),
- Konservierungsstoffe (z.B. para-Hydroxybenzoesäure-Ester, Benzalkoniumchlorid, Benzylalkohol, Thiomersal),
- Süßungsmittel oder Geschmackkorrigientien (z.B. Saccharose, Sorbit, Süßstoffe wie etwa Saccharin-Natrium und Cyclamat, Aromen, Ethylvanillin).

Die erfindungsgemäß einzusetzende zweite Mischung B) wird vorzugsweise mindestens teilweise durch ein Verfahren zur Feuchtgranulierung, besonders bevorzugt der Wirbelschichtgranulation, hergestellt. Dabei wird der erste Bestandteil B-i) mit mindestens einem zweiten Bestandteil B-ii) gemischt. Bei der Sprühgranulation, insbesondere Wirbelschichtgranulation bzw. Wirbelschichtgranulierung, handelt es sich um ein, dem Fachmann auf dem Gebiet der Pharmazeutischen Technologie bekanntes und übliches Verfahren mit breitem Einsatzgebiet und ist daher, insbesondere mit Blick auf Standardliteratur auf diesem Gebiet, dem Fachmann bekannt.

Erfindungsgemäß bevorzugt wird eine erste Komponente B-i) durch Sprühgranulation, vorzugsweise Wirbelschichtgranulation hergestellt, wobei die vorzugsweise miteinander gemischten Komponenten B-i-a) und B-i-b) (zusammen) mit einer Komponente B-i-c) enthaltenden Lösung besprüht werden.

Erfindungsgemäß vorteilhaft sind mindestens teilweise durch Sprühgranulation, insbesondere Wirbelschichtgranulation, erhaltene bzw. erhältliche erfindungsgemäß einzusetzende zweite Mischungen B) mit einer Größe von höchstens 5 mm, vorzugsweise von höchstens 2,5 mm, besonders bevorzugt von höchstens 1,25 mm.

Als "Feuchtgranulate" werden, was dem Fachmann auf dem Gebiet der Pharmazeutischen Technologie aus dem allgemeinen Fachwissen bekannt ist, Granulate bezeichnet, die sich erdfeucht anfühlen. Nach einem allgemeinen Herstellungsprinzip werden Pulver nach Aggregation durch Anlösen oder mittels Klebstofflösung derart zerteilt, dass Granulatkörner entstehen. Feuchtgranulate werden unterteilt in Krustengranulate und Klebstoffgranulate (auch: Bindemittelgranulate). Bei der Herstellung von Krustengranulaten wird mit einer Flüssigkeit befeuchtet, die die Pulverpartikel teilweise löst bzw. anlöst. Wenn die konzentrierte Flüssigkeit später trocknet, kristallisiert die oberflächlich gelöste Substanz unter Bildung von Feststoffbrücken aus. Dabei verkrusten die Primärteilchen zu Granulatkörnern. Klebstoffgranulate entstehen, wenn Lösungen aus makromolekularen Stoffen zum Befeuchten und Binden verwendet werden. Festkörperbrücken aus erhärtetem Klebstoff festigen die Agglomerate nach dem Trocknen. Feuchtgranulate werden regelmäßig als Pressgranulat hergestellt und durch ein Sieb gepresst. Insbesondere unterscheidet der Fachmann je nach Herstellungsverfahren zwischen Pressgranulaten, Schüttelgranulaten und Lochscheibengranulaten. Den diesen Arten von Granulaten zugrundliegenden Verfahren ist gemeinsam, dass eine feuchte Pulvermasse manuell und/oder maschinell durch ein oder mehrere Siebe und/oder ein oder mehrere Lochscheiben gepresst wird.

Alternativ sind Feuchtgranulate auch als Produkte eines Wirbelschichtgranulationsverfahrens erhältlich. Wirbelschichtgranulationsverfahren sind dem Fachmann auf dem Gebiet der Pharmazeutischen Technologie bekannt und sind unter Heranziehung von allgemeinem Fachwissen auf dem Gebiet ohne Weiteres durchführbar.

"Mischen" oder Vermischen im Sinne der vorliegenden Erfindung beschreibt das Vereinigen von Verbindungen, Komponenten oder Mischungen, sodass eine möglichst gleichmäßige Mischung, also Homogenität der Mischung, erreicht wird. Für das Vermischen ist es notwendig, dass eine Relativbewegung partieller Volumenelemente der zu vermischenden Verbindungen, Komponenten oder Mischungen zueinander vorhanden ist. Diese kann insbesondere durch von außen eingetragene, mechanische Energie zustande kommen. Ziel jeder Mischung ist es, eine möglichst gleichmäßige Verteilung aller Komponenten zu erhalten. Je geringer die Varianz der festgestellten Konzentration im Vergleich mit der Zusammensetzung der Gesamtmischung, desto besser ist die Mischgüte. Außerdem kann es zu Volumenkontraktionen bzw. -zunahmen kommen. Bedeutsam ist zudem die Auswahl des Mischers, um unterschiedliche Eigenschaften der zu mischenden Feststoffe auszugleichen. Typischerweise verwendet werden diffusives Mischen (z.B. Mischanlagen, die die Partikel durch Rotation bewegen, insbesondere Trommel-, Doppelkonus- und V-Mischer), Mischen durch Scherkräfte (basierend auf Gleitzonen zwischen dem Pulver; z.B. Rotormischer), und konvektives Mischen (basierend auf Zirkulationsmustern innerhalb der Pulvermenge; z.B. Band-, Paddel- oder Schaufelmischer). Neben der Homogenität einer Mischung, spielen auch Anforderungen an Sicherheit und Hygiene eine Rolle, insbesondere im Bereich der pharmazeutischen Technologie und im Lebensmittelsektor. Gängige Mischertypen sind unter anderem Schaufel-, Mulden-, Planeten-, Reib-, Schnecken-, Walzen-, Schleuder-, Trommel-, Konus-, Taumel-, Kreisel-, Kühl-, Vakuum- und Schwerkraftmischer (zu weiteren bekannten Verfahren siehe "Mischen von Feststoffen - Prinzipien, Verfahren, Mischer", Springer-Verlag, R. Weinekötter, H. Gericke, 1995).

Als "Körpertemperatur" wird im Sinne der vorliegenden Erfindung die Körperkerntemperatur, d.h. die Temperatur des Inneren des Thorax, des Abdomens und des Kopfes, verstanden. Je nach Außentemperatur, Alter und Tageszeit liegt die Körpertemperatur eines (physiologisch gesunden) Menschen in einem Bereich von 35 °C bis 38 °C, vorzugsweise im Bereich von 36,3 °C bis 37,4 °C. Letzterer Bereich wird üblicherweise auch als Normaltemperatur (afebril) bezeichnet. Im Rahmen der vorliegenden Erfindung bedeutet "Körpertemperatur" vorzugsweise eine Temperatur im Bereich von 35 bis 38 °C, weiter bevorzugt von 36,3 °C bis 37,4 °C, besonders bevorzugt von 37 °C. D.h., besonders bevorzugt gilt, dass für ein erfindungsgemäßes Verfahren bzw. eine erfindungsgemäße Schichttablette, dass Mg²⁺-Ionen aus der ersten Schicht gegenüber Mg²⁺-Ionen aus der zweiten Schicht bei einer Temperatur von 37 °C unter atmosphärischem Druck in Wasser schneller freigesetzt werden.

In einer bevorzugten Ausführungsform des erfindungsgemäßen Verfahrens handelt es sich um ein Verfahren zur Herstellung einer Schichttablette, wobei die erste Mischung zur Herstellung der ersten Schicht die folgenden Bestandteile enthält oder aus den folgenden Bestandteilen besteht:
A-i) Magnesiumhydroxid und/oder Magnesiumcarbonat,
A-ii) Cyanocobalamin und/oder Riboflavin,
A-iii) Magnesiumcarbonat,
A-iv) quervernetzte Natrium Carboxymethylcellulose, amorphe hochdisperse Kieselsäure,
A-v) Eisenoxid, und
A-vi) Calciumstearat
oder weiterhin bevorzugt um ein Verfahren zur Herstellung einer Schichttablette, wobei die zweite Mischung zur Herstellung der zweiten Schicht die folgenden Bestandteile enthält oder aus den folgenden Bestandteilen besteht:
B-i) mindestens einer ersten Komponente, umfassend oder bestehend aus:
   B-i-a) Trimagnesiumdicitrat 9-Hydrat,
   B-i-b) Cellulosepulver, und
   B-i-c) Trägerstoffen zur Feuchtgranulierung ausgewählt aus der Gruppe, bestehend aus Alginaten, Hydroxypropylmethylcellulose, und Sorbit enthaltendes Pulver
      und
B-ii) mindestens einer zweiten Komponente, umfassend oder bestehend aus:
   B-ii-a)Thiaminmononitrat und/oder Pyridoxinhydrochlorid, und optional
   B-ii-b) Calciumstearat.

Erfindungsgemäß sind die bevorzugten Ausführungsformen miteinander kombinierbar. Insbesondere betrifft das erfindungsgemäße Verfahren ein Verfahren zur Herstellung einer Schichttablette, wobei die erste Mischung zur Herstellung der ersten Schicht die vorausgehend genannten bevorzugten Bestandteile enthält oder aus ihnen besteht, das kombiniert wird mit einem Verfahren zur Herstellung einer Schichttablette, wobei die zweite Mischung zur Herstellung der zweiten Schicht die vorausgehend genannten bevorzugten Bestandteile enthält oder aus ihnen besteht.

Eine, mehrere oder sämtliche der folgenden Stoffe werden in den erfindungsgemäßen Verfahren bevorzugt verwendet bzw. sind bevorzugte Bestandteile der erfindungsgemäßen Schichttabletten.
- quervernetzte Natrium Carboxymethylcellulose; Holz-basiert; ohne gentechnisch veränderte Inhalte; höchstens 6 % Wassergehalt; als Supersprengmittel; (z.B. Solutab® A-IP (ROQUETTE)).
- hochreine und amorphe hochdisperse Kieselsäure; niedrige Restfeuchte; geschmacksneutral; als Fließregulierungsmittel; zur Verbesserung von Härte und Abrieb; (z.B. Aerosil® 200 Pharma (Evonik Resource Efficiency GmbH)).
- Tablettierhilfsmittel; (z.B. Tablettierhilfsmittel K (Merck KGaA, Cellulosepulver)).
- Hydroxypropylmethylcellulose; entsprechend Hypromellose 2910 des European Pharmacopoeia (Ph. Eur.) und United States Pharmacopeia (USP); Viskosität: 2,0 bis 6,0 mPa s als 2 %-ige wässrige Lösung bei 20 °C; Methoxyl-Substitution: 28 bis 30; Hydroxypropoxyl-Substitution: 7 bis 12; als Gelbildner, Verdickungsmittel, Filmbildner für Coatings, vorzugsweise um den Geruch und Geschmack von Wirkstoffen in Tabletten zu maskieren und/oder um Medikamente und Nahrungsergänzungsmittel vor Oxidation zu schützen und/oder um Tabletten oder Kapseln farblich zu überziehen, als Bindemittel für die Tablettierung und Trockengranulation, und/oder als Matrixbildner; (z.B. Mantrocel® E5 (GUSTAV PARMENTIER GmbH)).
- Sorbit enthaltendes Pulver; ca. 60 % Sorbit-Gehalt; unter anderem als nicht-kariogenes Süßungsmittel in Pulverform, Tablettierhilfsmittel, Füllmittel, Feuchthaltemittel, und/oder Stabilisierungsmittel; (z.B. Neosorb® P30/60 (ROQUETTE)).
- Hydroxypropyl Methylcellulose bzw. Hypromellose; als Filmbildner zur Herstellung von Filmüberzügen, insbesondere magensaftlöslichen Filmüberzügen, als Bindemittel in der Granulation und/oder als Porenbildner in Kombination mit retardierenden, wasserunlöslichen Überzügen; (z.B. Pharmacoat® (Shin-Etsu Chemical Co. Ltd.)).
- Polyethylene Glycol; durchschnittliche molare Masse im Bereich von circa 200 g/mol bis circa 8000 g/mol; entsprechend circa 4 bis circa 180 Ethylenoxid-Einheiten; als Hilfsstoff, als viskositätssenkende Stoffe, als Schmiermittel, als Feuchthaltemittel, und/oder als Bindemittel, insbesondere in der Granulation; (z.B. Lipoxol® (Sasol Performance Chemicals)).
- Mischung aus einer oder mehreren Hydroxypropylmethylcellulosen, einer oder mehreren mikrokristallinen Cellulosen und einem oder mehreren acetylierten Monoglyceriden; als Mittel zum Befilmen, zum Schutz aktiver Inhaltsstoffe gegen Feuchtigkeit, zur Verbesserung der physikalischen Eigenschaften wie beispielsweise Härte oder Brüchigkeit; (z.B. Sepifilm™ (SEPPIC S.A.)).

"Cellulose" bzw. "Cellulosederivate" werden im technischen Gebiet der vorliegenden Erfindung regelmäßig unter anderem verwendet als Verdickungsmittel, als Trägerstoff oder Füllstoff, als Trennmittel, als Überzugsmittel, und/oder Schaummittel. Als Lebensmittelzusatzstoff tragen "Cellulosen" die Bezeichnungen E 460 bis E 466. Insbesondere gilt dabei die Zuordnung: E 460i (Mikrokristalline Cellulose), E 460ii (Cellulosepulver), E 461 (Methylcellulose), E 463 (Hydroxypropylcellulose), E 464 (Hydroxypropylmethylcellulose), E 465 (Ethylmethylcellulose), E 466 (Carboxymethylcellulose).

Die hierin beschriebenen erfindungsgemäß einzusetzenden Bestandteile A-i) bis A-vi) bzw. B-i) und B-ii) bewirken ein vorteilhaftes Freisetzungsprofil des enthaltenen Magnesiums der durch das Verfahren erhaltenen Schichttabletten gegenüber vorbekannten Schichttabletten, wodurch die vorangehend genannte technische Aufgabe vorteilhaft gelöst wird.

Des Weiteren haben die Erfinder überraschend herausgefunden, dass die Verwendung von Eisenoxid als Bestandteil der erfindungsgemäßen ersten Mischung A) ein besseres Verpressen der erfindungsgemäßen Schichttabletten erlaubt, und damit eine weitere zusätzliche zugrundeliegende Aufgabe, die Ermöglichung leichter Verarbeitbarkeit bzw. Durchführung der erfindungsgemäßen Verfahrensschritte, vorteilhaft löst.

Des Weiteren haben die Erfinder überraschend herausgefunden, dass die Verwendung von Trimagnesiumdicitrat 9-Hydrat als Bestandteil der erfindungsgemäßen ersten Mischung A) ein besseres Verpressen der erfindungsgemäßen Schichttabletten erlaubt, und damit einen weiteren der zugrundeliegenden Aufgabe, die leichte Verarbeitbarkeit bzw. Durchführung der erfindungsgemäßen Verfahrensschritte, vorteilhaft löst.

Besonders bevorzugt ist ein erfindungsgemäßes Verfahren zur Herstellung einer Schichttablette, wobei die erste Mischung A) in Schritt II) vorgepresst wird.

"Vorpressen" (oder auch Anpressen) beschreibt einen ersten bzw. nicht finalen Pressschritt im erfindungsgemäßen Verfahren zur Herstellung einer erfindungsgemäßen Schichttablette, wobei ein Schichttabletten-Vorpressling hergestellt bzw. erzeugt wird, bei dem nicht die endgültige Härte der erfindungsgemäßen Schichttablette erreicht wird. Ein "Schichttabletten-Vorpressling" im Sinne der vorliegenden Erfindung ist demnach das Zwischenerzeugnis nach dem Schritt II) bzw. durch Durchführen des Schritts II) des erfindungsgemäßen Verfahrens erhalten wird. Ebenso ist es möglich, das Vorpressen in einen oder mehreren Schritten durchzuführen. Dem gegenüber beschreibt das "Verpressen" bzw. das "Gemeinsame Verpressen" den finalen Pressschritt im erfindungsgemäßen Verfahren zur Herstellung einer erfindungsgemäßen Schichttablette, bei dem letztere ihre endgültige Härte erhält. Demnach setzt sich das (Gesamt-)Pressen des erfindungsgemäßen Verfahrens zusammen aus mindestens einem Schritt des Vorpressens bzw. Anpressens und einem abschließenden Schritt des Verpressens; d.h. im Sinne der vorliegenden Erfindung beschreibt des Verpressen vorzugsweise den finalen Pressschritt.

Überraschenderweise haben die Erfinder gefunden, dass die erfindungsgemäß durch dieses Verfahren erhaltenen Schichttabletten, bei denen die erste Mischung A) in Schritt II) vorgepresst wird, überlegene Eigenschaften im Sinne ihrer physikalischen Eigenschaften aufweisen.

Besonders bevorzugt ist ein erfindungsgemäßes Verfahren zur Herstellung einer Schichttablette ein Verfahren, wobei Schritt II) bis zum Erreichen einer Härte der Mischung A) oder B) in mindestens einer räumlichen Richtung des Schichttabletten-Vorpresslings im Bereich von 5 - 15 N, vorzugsweise im Bereich von 6 - 13 N, besonders bevorzugt im Bereich von 7 - 11 N durchgeführt wird.

Die "Härte", die im Sinne der vorliegenden Erfindung nach den Schritten der An- bzw. Vorpressens und Verpressens bestimmt werden kann, ist einer der geeigneten Parameter zur Bestimmung der mechanischen Festigkeit. Prüfungen mechanischer Festigkeit erstrecken sich unter anderem auf Verschleiß-, Roll-, Schüttel-, Schlag-, Reib-, Druck-, und Biegefestigkeit. Vorzugsweise beschreibt die Härte im Sinne der vorliegenden Erfindung die Biege- und Druckfestigkeit. Genaueres zur Härtemessung im Sinne der vorliegenden Erfindung ist den Beispielen zu entnehmen. Biegefestigkeit bezeichnet den Widerstand, den eine Tablette einem auf ihrer Ober- oder Unterseite wirkenden Druck leistet, wobei die der Druckstelle gegenüberliegende Seite nicht unterstützt wird. Druckfestigkeit ist der Widerstand einer Tablette gegen eine diametrisch wirkende Kraft, bei welcher die Tablette zerbricht. Die dem Fachmann bekannten Geräte zur Messung der Druckfestigkeit werden häufig auch als Härtetester bezeichnet. Zu beachten ist, dass Härte als die Widerstandskraft der Oberfläche eines Festkörpers gegen das Eindringen eines spitzen oder kegelförmigen Prüfkörpers verstanden wird. Härteprüfungen sind somit definitionsgemäß Oberflächenfestigkeitsprüfungen. Dem Fachmann auf dem Gebiet der Pharmazeutischen Technologie sind und unter Heranziehung von allgemeinem Fachwissen ohne Weiteres geeignete Messprinzipien bzw. Messmethoden bekannt. Als Grundlage für die Bestimmung der Härte dienen vorzugsweise Geräte ausgewählt aus der Gruppe umfassend oder bestehend aus einem Stokes-Monsato-Härtetester, einem Strong-Cobb-Tabletten-Härtetester, einem Pfizer-Tabletten-Härtetester, einem Shore-Härtetester, einem Erweka-Bruchfestigkeitsmesser und einem Heberlein-Bruchfestigkeitsmesser. Der Bruchwert wird üblicherweise in Newton (N) oder Kilopond (kp) angegeben.

Die Prüfung der Bruchfestigkeit von Tabletten dient dazu, unter definierten Bedingungen die Bruchfestigkeit von Tabletten zu bestimmen. Bei der Prüfung wird die Kraft gemessen, die notwendig ist, um die Tabletten durch Druck zu zerbrechen. Das Gerät besteht aus 2 Backen, die sich gegenüberstehen. Einer der Backen bewegt sich auf den anderen zu. Die Backenflächen sind flach und größer als die Kontaktzone zur Tablette sowie senkrecht zur Bewegungsrichtung angeordnet. Das Gerät wird mit einem System, das eine Genauigkeit von etwa 1 Newton aufweist, kalibriert. Die Tablette wird zwischen die Backen gelegt, wobei gegebenenfalls die Form, die Bruchrille und die Prägung berücksichtigt werden. Bei jeder Messung wird die Tablette in gleicher Weise in Bezug auf die Richtung der Kraft orientiert. Die Prüfung wird an 10 Tabletten durchgeführt. Tablettenfragmente müssen vor jeder Prüfung entfernt werden. Der Mittelwert und die Extremwerte der gemessenen Kräfte, ausgedrückt in Newton, werden als Ergebnis angegeben. Auf den Gerätetyp und, falls zutreffend, auf die Ausrichtung der Tabletten wird hingewiesen (Ph. Eur. 2.9.8, Ph. Eur. 8. Ausgabe, Grundwerk 2014).

Die Erfinder haben überraschend festgestellt, dass die erfindungsgemäß durch dieses Verfahren erhaltenen Schichttabletten, bei denen der Schichttabletten-Vorpressling in der vorausgehend beschriebenen Art und Weise bis zum Erreichen einer definierten Härte vorgepresst wird, überlegene Eigenschaften im Sinne ihrer physikalischen Eigenschaften aufweisen. Insbesondere haben die Erfinder festgestellt, dass die vorausgehend spezifizierte Härte der erfindungsgemäßen Schichttabletten-Vorpresslinge ein vorteilhaftes Befilmen der daraus erhaltenen Schichttabletten, d.h. beispielsweise ohne wesentlichen Abrieb von oder Zerbröseln der erfindungsgemäßen Schichttablette erlaubt.

Besonders bevorzugt ist ein erfindungsgemäßes Verfahren zur Herstellung einer Schichttablette, wobei Schritt IV) bis zum Erreichen einer (endgültigen) Härte der Schichttablette in mindestens einer räumlichen Richtung der Schichttablette, vorzugsweise, falls vorhanden, in Längsrichtung der Schichttablette, im Bereich von 200 - 250 N, vorzugsweise im Bereich von 210 - 240 N, besonders bevorzugt im Bereich von 220 - 230 N durchgeführt wird.

Die "endgültige Härte" ist zu verstehen als die Härte einer erfindungsgemäßen Schichttablette bzw. des Erzeugnisses nach Abschluss eines erfindungsgemäßen Verfahrens zur Herstellung einer Schichttablette, d.h. nach dem finalen Pressschritt.

Die Erfinder haben überraschend festgestellt, dass die erfindungsgemäß durch dieses Verfahren erhaltenen Schichttabletten, bei denen die Schichttablette in der vorausgehend beschriebenen Art und Weise bis zum Erreichen einer definierten Härte verpresst wird, überlegene Eigenschaften im Sinne ihrer physikalischen Eigenschaften aufweisen. Insbesondere haben die Erfinder festgestellt, dass die vorausgehend spezifizierte Härte der erfindungsgemäßen Schichttabletten ein vorteilhaftes Befilmen, d.h. beispielsweise ohne wesentlichen Abrieb von oder Zerbröseln der erfindungsgemäßen Schichttablette erlaubt.

Weiterhin ist besonders bevorzugt ein erfindungsgemäßes Verfahren zur Herstellung einer Schichttablette wie hierin beschrieben, wobei Schritt II) und/oder Schritt IV) das Erzeugen mindestens einer oder mehrerer Bruchrillen auf mindestens einer Seite des Schichttabletten-Vorpresslings oder der Schichttablette umfasst, oder, umfassend mindestens einen zusätzlichen Schritt des Erzeugens mindestens einer oder mehrerer Bruchrillen auf mindestens einer Seite des Schichttabletten-Vorpresslings oder der Schichttablette.

Eine "Bruchrille" im Sinne der vorliegenden Erfindung ist eine Bruchkerbe, die sich über mindestens eine Fläche der erfindungsgemäßen Schichttablette erstreckt bzw. durch das erfindungsgemäße Verfahren eingebracht wird, um das Zerteilen der Schichttablette zu erleichtern oder eine Schmuckkerbe, die unter anderem der Identifizierung und/oder Unterscheidung von Tabletten dient.

Besonders bevorzugt ist ein erfindungsgemäßes Verfahren zur Herstellung einer Schichttablette, wobei das Befilmen der Schichttablette nach Schritt IV) durch Besprühen mit einer Film-Sprühlösung und/oder Beschichtung in einer Beschichtungsapparatur erfolgt.

"Befilmen" im Sinne der vorliegenden Erfindung beschreibt das Überziehen von peroralen oder oralen Darreichungsformen (Tabletten, Pillen, Dragees, etc.), die vorzugsweise geschluckt, gelutscht oder gekaut werden, mit einer Überzugsschicht. Dieses Verfahren führt zur Bildung eines Films bzw. einer Beschichtung auf der Oberfläche der zu beschichtenden Darreichungsform. Vorzugsweise findet ein Befilmen statt mittels einem oder mehreren Verfahren ausgewählt aus der Gruppe, bestehend aus "klassischem" Dragieren in rotierenden Kesseln unter manueller und/oder automatisierter (anteilsweiser) Zugabe einer Dragierflüssigkeit, Aufsprühen einer Dragierflüssigkeit, dem Wirbelbettverfahren oder der Kesselfilmlackierung (R. Voigt, Pharmazeutische Technologie, 10. Auflage, Deutscher Apotheker Verband Stuttgart). Letztere Verfahren sind dem Fachmann auf dem Gebiet der vorliegenden Erfindung aus dem allgemeinen Fachwissen bekannt. Durchgeführt wird ein Befilmen regelmäßig aufgrund einer oder mehrerer Wirkungen bzw. Eigenschaften, die ausgewählt ist bzw. sind aus der Gruppe, bestehend aus Verdecken von unangenehmem Geschmack bzw. Geruch, Schützen eines Wirkstoffs oder mehrerer Wirkstoffe gegen äußere Einflüsse (Luftsauerstoff, Luftfeuchtigkeit, UV-Strahlung), Stärken der mechanischen Widerstandsfähigkeit gegen mechanische Beanspruchung, vorzugsweise Bereitstellen eines weitestgehenden Schutzes des Konsumenten vor Bestandteilen des zu befilmenden Produkts, die Mund oder Magenschleimhaut reizen, Erleichtern des Schluckvorgangs durch glattere Oberflächen und den Fortfall von Kanten, Erzielen einer psychologischen Wirkung hinsichtlich der Einnahme des Produkts beim Konsumenten, Reduzieren der Verwechslungsgefahr des Produkts mit anderen Präparaten, insbesondere bei (gleichzeitiger) und Verdecken von aus verfahrenstechnischen Gründen nicht einheitlichen Oberflächendesigns.

Weiterhin besonders bevorzugt ist ein erfindungsgemäßes Verfahren zur Herstellung einer Schichttablette, wobei das Gewichtsverhältnis der ersten Mischung A) zur zweiten Mischung B) im Bereich von 1 : 3 bis 3 : 1, vorzugsweise im Bereich von 1 : 1,5 bis 1,5 : 1, besonders bevorzugt im Bereich von 1 : 1,1 bis 1,1 : 1 liegt, und/oder wobei das Gesamtgewicht der Schichttablette im Bereich von 10 mg bis 2500 mg, vorzugsweise im Bereich von 700 mg bis 2000 mg, besonders bevorzugt im Bereich von 1400 mg bis 1800 mg liegt.

Die Bestimmung des Gehalts an Magnesium bzw. Mg²⁺-Ionen in den erfindungsgemäßen Schichttabletten erfolgt vorzugsweise mit Hilfe der dem Fachmann bekannten Prüfung auf Gleichförmigkeit einzeldosierter Arzneiformen gemäß dem Europäischen Arzneibuch (Ph. Eur.), mit dem Zweck, die Gleichmäßigkeit der Dosierung von abgeteilten Arzneiformen zu bestimmen. Anhand von höchstens 30, nach dem Stichprobenverfahren entnommenen, Einheiten, wird ermittelt, ob der Gehalt innerhalb der zu tolerierenden Grenzwerte liegt. Die im Folgenden spezifizierten Gehalte an Magnesium bzw. Mg²⁺-Ionen sind zu verstehen als Nennwert bzw. Sollwert.

Die Erfinder haben überraschend festgestellt, dass eine erfindungsgemäße Schichttablette der vorausgehend genannten Gewichtsverhältnisse der ersten Mischung A) zur zweiten Mischung B) bzw. des vorausgehend beschriebenen Gesamtgewichts die zugrundeliegende technische Aufgabe besonders vorteilhaft löst.

Besonders bevorzugt ist weiterhin ein erfindungsgemäßes Verfahren zur Herstellung einer Schichttablette, wobei das Gewichtsverhältnis des Films C), falls vorhanden, zur Summe aus der ersten Mischung A) und der zweiten Mischung B) im Bereich von 1 : 80 bis 1 : 10, vorzugsweise im Bereich von 1 : 40 bis 1 : 25, besonders bevorzugt im Bereich von 1 : 35 bis 1 : 30 liegt.

Weiterhin besonders bevorzugt ist ein erfindungsgemäßes Verfahren zur Herstellung einer Schichttablette, wobei die Schichttablette einen Gehalt an Magnesium bzw. Mg²⁺-Ionen im Bereich von 100 mg bis 1000 mg, vorzugsweise im Bereich von 200 mg bis 750 mg, besonders bevorzugt im Bereich von 250 mg bis 500 mg aufweist.

Besonders bevorzugt ist weiterhin ein erfindungsgemäßes Verfahren zur Herstellung einer Schichttablette, wobei das Gewichtsverhältnis des durch die erste Mischung A) bereitgestellten Gehalts an Magnesium bzw. Mg²⁺-Ionen zu dem durch die zweite Mischung B) bereitgestellten Gehalt an Magnesium bzw. Mg²⁺-Ionen im Bereich von 1 : 6 bis 6 : 1, vorzugsweise im Bereich von 1 : 4 bis 4 : 1, besonders bevorzugt im Bereich von 1 : 3 bis 3 : 1 liegt.

Weiterhin besonders bevorzugt ist ein erfindungsgemäßes Verfahren zur Herstellung einer Schichttablette, wobei das Gewichtsverhältnis des durch die erste Mischung A) bereitgestellten Gehalts an Magnesium bzw. Mg²⁺-Ionen zu dem durch die zweite Mischung B) bereitgestellten Gehalt an Magnesium bzw. Mg²⁺-Ionen im Bereich von 1 : 6 bis 6 : 1, vorzugsweise im Bereich von 1 : 4 bis 4 : 1, besonders bevorzugt im Bereich von 1 : 3 bis 3 : 1 liegt.

Besonders bevorzugt ist weiterhin ein erfindungsgemäßes Verfahren zur Herstellung einer Schichttablette, wobei die erste Mischung A) mindestens einen Hilfsstoff enthält und/oder die zweite Mischung B) unabhängig davon mindestens einen Hilfsstoff enthält, wobei der mindestens eine Hilfsstoff bzw. die Hilfsstoffe, unabhängig voneinander, ausgewählt ist bzw. sind aus der Gruppe, bestehend aus Süßungsmitteln, vorzugsweise Laktose, Saccharose oder Sorbit; Säureregulatoren, vorzugsweise Kaliumtartrat; Trennmitteln, vorzugsweise Salzen, insbesondere Calciumsalzen, von Speisefettsäuren, vernetzter Natriumcarboxymethylcellulose oder Siliciumdioxid; Säuerungsmitteln, vorzugsweise Zitronensäure; Aromen, vorzugsweise natürlichen Aromen; Füllstoffen, vorzugsweise Hydroxypropylcellulose; weiteren Citratsalzen, vorzugsweise Natriumcitrat, Kaliumcitrat, Kalium-Natrium-Hydrogencitrat, Calcium-Natrium-Hydrogencitrat, Kupfercitrat und/oder Eisencitrat; Calciumcarbonat, Calciumlactat, Natriumhydrogencarbonat; L-Carnitin; Maltodextrin; Molybdän, vorzugsweise Natriummolybdat; Chrom, vorzugsweise Chromchlorid; und Selen, vorzugsweise Natriumselenit, Natriumselenat oder Selenhefe; vorzugsweise wobei der mindestens eine Hilfsstoff ausgewählt ist aus der Gruppe, bestehend aus mikrokristalliner Cellulose, Hydroxypropylcellulose, Sorbit, Stearinsäure und Alginat.

Weiterhin besonders bevorzugt ist ein erfindungsgemäßes Verfahren zur Herstellung einer Schichttablette, wobei die erste Mischung A) und/oder die zweite Mischung B) einen oder mehrere Farbstoffe und/oder Pigmente in einer Menge enthält bzw. enthalten, sodass die erste Schicht von der zweiten Schicht der Schichttablette visuell durch das menschliche Auge, vorzugsweise auch bei Farb-Fehlsichtigkeiten bzw. nicht vollständig funktionsfähiger Polychromasie, unterschieden werden kann.

Besonders bevorzugt ist weiterhin ein erfindungsgemäßes Verfahren zur Herstellung einer Schichttablette, wobei der Film C) besteht aus oder enthält:
- eine, mehrere oder sämtliche Verbindungen ausgewählt aus der Gruppe, bestehend aus Hydroxypropylmethylcellulosen oder Hypromellose, Polyethylene Glycol, Talkum, mikrokristallinen Cellulosen und acetylierten Monoglyceriden, und optional
- einen Geschmacksstoff.

Weiterhin besonders bevorzugt ist ein erfindungsgemäßes Verfahren zur Herstellung einer Schichttablette, wobei die in Schritt I) bereitgestellten Mischungen A) und/oder B) unabhängig voneinander eine Feuchte im Bereich von 0,1 bis 10 %, vorzugsweise im Bereich von 0,5 bis 5 %, besonders bevorzugt im Bereich von 1 bis 2 %, bezogen auf das Gesamtgewicht der jeweiligen Mischung A) oder B), aufweisen.

"Feuchte" im Sinne der vorliegenden Erfindung beschreibt den Gehalt an Feuchtigkeit in den erfindungsgemäßen Mischungen A) und B) in Gew.-%, bezogen auf das Gewicht dieser Mischungen A) und B). Dabei unterscheidet sich regelmäßig der Gehalt an Feuchtigkeit der erfindungsgemäßen Mischung A) vom Gehalt an Feuchtigkeit der erfindungsgemäßen Mischung B). Der Feuchtigkeits- bzw. Wassergehalt von Pulvern beeinflusst unter anderem die Fließfähigkeit, Agglomerationstendenz und Mischbarkeit von Haufwerken. Diese Eigenschaften spielen eine Rolle für die weitere Bearbeitung, Lagerung, und Verwendung daraus abgeleiteter Erzeugnisse. Regelmäßig wird die Feuchte von Tabletten bestimmt mittels Gravimetrie (Rückwaage im Trockenschrank-Verfahren), Karl-Fischer-Titration oder leitfähigkeits-basierten elektronischen Messverfahren. Vorzugsweise erfolgt die Bestimmung der Feuchte durch ein Infrarot (IR) Messverfahren mittels Heizstrahler (Sartorius Moisture Analyzer). Weiteres zur Feuchtebestimmung im Sinne der vorliegenden Erfindung ist den Beispielen zu entnehmen.

Die Erfinder haben überraschend festgestellt, dass sich die vorausgehend genannten Werte der erfindungsgemäßen Feuchte besonders vorteilhaft auf das erfindungsgemäße Verfahren zur Herstellung einer Schichttablette auswirken und die zugrundeliegende technische Aufgabe besonders vorteilhaft lösen können. Insbesondere wirkt sich die vorausgehend erläuterte Feuchte bzw. Restfeuchte innerhalb des spezifizierten Bereichs vorteilhaft auf die Vorpressbarkeit und/oder Verpressbarkeit der erfindungsgemäßen Schichttabletten bzw. der Schichttabletten-Vorpresslinge aus.

Besonders bevorzugt ist weiterhin ein erfindungsgemäßes Verfahren zur Herstellung einer Schichttablette, wobei in der Wirbelschichtgranulation als Verfahren zur Feuchtgranulierung, falls verwendet, die Bestandteile der einen, mehreren oder sämtlichen Komponenten B-ii) auf die Bestandteile der einen bzw. mehreren optional bereits beschichteten Komponenten B-i) einer, mehrere oder sämtliche der folgenden Parameter verwendet werden:
- Zulufttemperatur im Bereich von 20 bis 80 °C, vorzugsweise im Bereich von 25 bis 60 °C, besonders bevorzugt im Bereich von 30 bis 40 °C,
- Sprühluftdruck im Bereich von 1 bis 7 bar, vorzugsweise im Bereich von 1 bis 4 bar, besonders bevorzugt im Bereich von 1,25 bis 1,75 bar,
- Rüttelzeit im Bereich von 5 bis 25 s, vorzugsweise im Bereich von 6 bis 19 s, besonders bevorzugt im Bereich von 7 bis 17 s.

"Sprühgranulation", insbesondere Wirbelschichtgranulation, ist dem Fachmann auf dem Gebiet der Pharmazeutischen Technologie bekannt und unter Heranziehung von allgemeinem Fachwissen auf dem Gebiet ohne Weiteres zugänglich. Bezüglich eine beispielhaften Auswahl der erfindungsgemäßen Prozessparameter sei an dieser Stelle insbesondere auf die Beispiele verwiesen.

Im Sinne der vorliegenden Erfindung wird Magnesium (Mg) bzw. werden Mg²⁺-Ionen durch die Anwesenheit von einem oder mehreren Mg- bzw. Mg²⁺-Ionen enthaltenden Salzen bereitgestellt. Vorzugsweise ist das bzw. sind die Salz(e) ausgewählt aus der Gruppe, bestehend aus Magnesiumoxid, Magnesiumhydroxid, Magnesiumcarbonat, Magnesiumaspartat, Magnesiummalat, Magnesiumgluconat, Magnesiumglycinat, Magnesiumorotat, Magnesiumacetat, Magnesiumascorbat, Magnesiumformiat, Magnesiumfumarat, Magnesiumglutamat, Magnesiumglycerophosphat, Magnesiumlactat, Magnesiumnitrat, Magnesiumoxalat, Magnesiumphosphat, Magnesiumpidolat, Magnesiumpropionat, Magnesiumtartrat, Magnesiumchlorid, Magnesiumsulfat, und Magnesiumcitraten bzw. Magnesiumdicitraten, insbesondere wasserfreies Trimagnesiumdicitrat (CAS 3344-18-1), und Trimagnesiumdicitrat Tetradecahydrat (CAS 6150-79-4). Trimagnesiumdicitrat 9-Hydrat bzw. Trimagnesiumdicitrat Nonahydrat (CAS 153531-96-5) ist eine chemische Verbindung aus der Gruppe der Magnesiumcitrate und dient im Sinne der vorliegenden Erfindung besonders bevorzugt zur Bereitstellung von Mg bzw. Mg²⁺-Ionen. Besonders bevorzugt im Sinne der vorliegenden Erfindung ist Trimagnesiumdicitrat 9-Hydrat.

Eine "Temperatur im Bereich" bzw. "Zulufttemperatur im Bereich" von einer Untergrenze bis zu einer Obergrenze im Sinne der vorliegenden Erfindung bedeutet, dass sich die Temperatur während des wesentlichen Teils der Reaktionsdauer innerhalb des spezifizierten Bereichs befindet. Darunter sind sowohl isotherme Reaktionsbedingungen als auch Reaktionsbedingungen mit Temperaturprofilen zu verstehen, bei denen sich die Temperatur im Wesentlichen innerhalb der spezifizierten Grenzen bewegt. Ein kurzzeitiges Verlassen des Temperaturbereichs für einen nicht wesentlichen Zeitraum steht dem nicht gegenüber.

Selbiges gilt analog für einen "Druck im Bereich" bzw. "Sprühluftdruck im Bereich" und eine "Rüttelzeit im Bereich" von einer Untergrenze bis zu einer Obergrenze.

Wenn nicht explizit anders erwähnt, wird das Verfahren bzw. werden die einzelnen, mehrere oder sämtliche Verfahrensschritte unabhängig voneinander bei Standardbedingungen, also Standardtemperatur und/oder Standardruck durchgeführt. Sofern nur Werte für einen der Parameter angegeben sind, gelten in diesem Fall vorzugsweise für den anderen Standardbedingungen.

Im Folgenden werden erfindungsgemäße Schichttabletten, erhalten oder erhältlich gemäß einem erfindungsgemäßen Verfahren zur Herstellung erfindungsgemäßer Schichttabletten beschrieben. Für bevorzugte Ausgestaltungen dieser Schichttabletten gilt aber grundsätzlich das oben im Zusammenhang mit einem erfindungsgemäßen Verfahren Gesagte entsprechend.

Ein weiterer Aspekt der vorliegenden Erfindung, der die zugrundeliegende Aufgabe vorteilhaft löst, betrifft demnach eine Schichttablette erhalten durch oder erhältlich gemäß einem erfindungsgemäßen Verfahren.

Für die in den erfindungsgemäßen Schichttabletten enthaltenen Verbindungen gelten weiterhin die obigen Ausführungen.

Vorzugsweise weist eine erfindungsgemäße Schichttablette eine äußere Form ausgewählt aus der Gruppe, bestehend aus dreieckig, viereckig, fünfeckig, sechseckig, achteckig, sonderförmig, rund, oval, oblong, zylinderförmig, torpedoförmig, kugelförmig, kegelförmig, pyramidenförmig und tropfenförmig auf.

Bevorzugt ist eine Schichttablette erhalten durch oder erhältlich durch ein erfindungsgemäßes Verfahren zur Herstellung wie hierin beschrieben, umfassend oder bestehend aus einer ersten, Mg²⁺-Ionen freisetzenden Schicht und einer zweiten, Mg²⁺-Ionen freisetzenden Schicht, und optional einem Film, wobei
- die erste Schicht die folgenden Bestandteile enthält oder aus den folgenden Bestandteilen besteht:
   A-i), A-ii), A-iii), A-iv), und optional A-v), und optional A-vi), wie jeweils hierin beschrieben,
- die zweite Schicht die folgenden Bestandteile enthält oder aus den folgenden Bestandteilen besteht:
   B-i) und B-ii), wie jeweils hierin beschrieben, und gegebenenfalls
- der Film die Bestandteile wie hierin beschrieben enthält oder daraus besteht.

Für die in den erfindungsgemäßen Schichttabletten enthaltenen Verbindungen gelten weiterhin die obigen Ausführungen.

Besonders bevorzugt ist eine erfindungsgemäße Schichttablette, wobei die Schichttablette ein Nahrungsergänzungsmittel, vorzugsweise in Form einer Tablette zur peroralen Einnahme ist.

"Perorale Einnahme" bedeutet im Sinne der vorliegenden Erfindung die Verabreichung über den Mund. Eine Substanz wird somit bei peroraler Einnahme geschluckt. Dabei kann eine Substanz, bzw. eine davon abgeleitete Zubereitung oder ein davon abgeleitetes Erzeugnis in flüssiger, fester, pastösen, oder sonstigen schluckbaren Form vorliegen. Zu den festen Zubereitungen gehören Tabletten, Kapseln, Granulate und Pulver zum Einnehmen. Die festen Formen sind meist besser haltbar und reduzieren weiterhin vorzugsweise den unangenehmen Geschmack eines Wirkstoffs oder einiger Wirkstoffe.

Weiterhin besonders bevorzugt ist eine erfindungsgemäße Schichttablette, wobei der Film C) die Schichttablette weitestgehend vollständig, vorzugsweise vollständig, besonders bevorzugt vollständig und gleichmäßig bedeckt.

Vorzugsweise ist eine erfindungsgemäße Schichttablette eine Schichttablette, die frei von einem, mehreren oder sämtlichen der Bestandteile ausgewählt aus der Gruppe bestehend aus Laktose, Iod, Gluten, Aromastoffen und Süßungsmitteln ist, vorzugsweise frei ist von Laktose, Iod und Gluten ist.

Ein weiterer Aspekt der vorliegenden Erfindung betrifft eine erfindungsgemäße Schichttablette zur Anwendung als Medikament.

Bevorzugt ist eine erfindungsgemäße Schichttablette zur Anwendung bei der Behandlung und/oder Vorbeugung einer oder mehrerer Stoffwechselerkrankungen und/oder einer oder mehrerer Mineralstoffmangelerkrankungen, wobei die Stoffwechselerkrankung(en) und/oder Mineralstoffmangelerkrankung(en) vorzugsweise ausgewählt ist bzw. sind aus der Gruppe, bestehend aus Hypomagnesiämie, Herzrhythmusstörungen, Migräne und Hypertonie.

Besonders bevorzugt ist eine erfindungsgemäße Schichttablette zur Anwendung wie hierin beschrieben, wobei mindestens eine unerwünschte Nebenwirkung durch die, die durch die erfindungsgemäße Schichttablette bedingte erhöhte Aufnahme von Magnesium in den menschlichen Körper hinsichtlich Stärke der mindestens einen Nebenwirkung und/oder Häufigkeit der mindestens einen Nebenwirkung verringert ist bzw. sind oder nicht auftreten, vorzugsweise wobei die mindestens eine Nebenwirkung ausgewählt ist bzw. sind aus der Gruppe, bestehend aus abführender Wirkung, weicher Stuhl, Durchfall und Müdigkeitserscheinungen.

Eine "Nebenwirkung" im Sinne der vorliegenden Erfindung ist eine schädliche und/oder unbeabsichtigte Reaktion, die insbesondere bei einem bestimmungsgemäßen Gebrauch auftreten. Vorzugsweise ist die Nebenwirkung, die eingeschränkt werden soll ausgewählt aus der Gruppe, bestehend aus abführender Wirkung, weichem Stuhl, Durchfall (Diarrhoe), und Müdigkeitserscheinungen; vorzugsweise ist die erfindungsgemäß einzuschränkende Nebenwirkung eine abführende Wirkung oder das Auftreten von Diarrhoe.

Eine hinsichtlich Stärke und/oder Häufigkeit "verringerte Nebenwirkung" ist zu verstehen als eine Verringerung hinsichtlich Stärke und/oder Häufigkeit der hierin genannten Nebenwirkungen einer erfindungsgemäßen Darreichungsform im Vergleich zur Darreichung derselben Menge eines Wirkstoffs unter denselben Voraussetzungen und Bedingungen in freier Form bzw. nicht gemäß der Erfindungsgemäßen. Allgemein sind Magnesiumpräparate, insbesondere auf Trimagnesiumdicitrat basierte Präparate, für ihre abführende Wirkung bekannt. Eine erfindungsgemäße verringerte Nebenwirkung oder unterdrückte Nebenwirkung ist dahingehend die weitestgehende Verringerung bzw. Unterdrückung der abführenden Wirkung durch einen oder mehrere Wirkstoffe, die in erfindungsgemäßen Schichttabletten enthalten sind.

### Kurzbeschreibung der Figuren:

Figur 1 zeigt eine Schichttablette 1 bestehend aus einer ersten, Mg²⁺-Ionen freisetzenden, Schicht 2, und einer zweiten, Mg²⁺-Ionen freisetzenden, Schicht 3.
Figur 2 zeigt eine Schichttablette 1 bestehend aus einer ersten, Mg²⁺-Ionen freisetzenden, Schicht 2, und einer zweiten, Mg²⁺-Ionen freisetzenden, Schicht 3, wobei die Schichttablette 1 eine Bruchrille 4 aufweist.
Figur 3 zeigt eine Schichttablette (1) bestehend aus einer ersten, Mg²⁺-Ionen freisetzenden, Schicht 2, und einer zweiten, Mg²⁺-Ionen freisetzenden, Schicht 3, wobei die Schichttablette 1 von einem Film oder einer Beschichtung 5 umhüllt ist.
Figur 4 zeigt eine Schichttablette 1 bestehend aus einer ersten, Mg²⁺-Ionen freisetzenden, Schicht 2, und einer zweiten, Mg²⁺-Ionen freisetzenden, Schicht 3, wobei die Schichttablette 1 eine Bruchrille 4 aufweist und von einem Film oder einer Beschichtung 5 umhüllt ist.

Der Ausdruck "mindestens eine/r/s" im Zusammenhang mit der Auswahl aus einer Gruppe, bestehend aus Elementen bzw. abgeschlossenen Aufzählung von Elementen versteht sich im Sinne des vorliegenden Textes als eine Auswahl von einem, mehrerer, oder sämtlichen Elementen aus der jeweiligen Gruppe, in beliebiger Kombination miteinander.

Im Folgenden wird die vorliegende Erfindung anhand von ausgewählten Beispielen näher erläutert, wobei die vorliegende Erfindung nicht hierauf beschränkt ist.

### Beispiele

Sofern nicht anders angegeben, beziehen sich sämtliche der folgenden Angaben auf das Gewicht.

### A) "erste Mischung"

Zur Herstellung der "ersten Mischung" werden die Rohstoffe (0,25 kg Cyanocobalamin 1% SD; 1,39 kg Riboflavin; 14,48 kg Solutab A-IP; 13,88 kg Magnesiumhydroxid DC MS 90) durch ein 1mm-Sieb in die Rührschüssel eines elektrischen Groß-Küchengeräts gegeben und darin mit einem geeigneten Anbauteil (z.B. Rührflügel) bis zur durch Umschaufeln optisch erkennbaren Homogenität vermischt, um eine "Vormischung" zu erhalten. Ebenso werden weitere Rohstoffe (162,00 kg Magnesiumhydroxid DC MS 90; 160,56 kg destabilisiertes Magnesiumcarbonat 90 S; 1,81 kg Eisenoxid (leuchtendrot); 1,50 kg Aerosil 200 Pharma; 4,24 kg Calciumstearat) gemeinsam mit 15,00 kg der vorausgehend hergestellten "Vormischung" durch ein 1,25mm-Sieb in den ELO-Container eines elektrischen Mischgeräts (hier Container-Mischer) gegeben und darin 15 Minuten bis zur optisch erkennbaren (Sichtfenster) Homogenität vermischt.

### B) "zweite Mischung"

1) Herstellung der Sprühlösung umfassend den bzw. die Bestandteil(e) B-i-c): Nisso HPC-SSL (2,50 kg) und Neosorb (5,91 kg) wurden in vorgelegtem kalten Wasser (23,16 kg) vollständig klar gelöst, oder vorzugsweise mit einem Rührwerk mit Rührscheibe eingerührt. Dabei wurde beobachtet, dass die Lösung erst über Nacht klar wurde. Die Herstellung der gesamten Wirbelschichtgranulat-Sprühlösung umfassend den bzw. die Bestandteil(e) B-i-c) wurde in sechs Einzelansätzen in den o.g. Mengenangaben durchgeführt.
2) Herstellung der Mischung umfassend die Bestandteile B-i-a) und B-i-b): Trimagnesiumdicitrat 9-hydrat Gran Lohm (B-i-a)) (314,91 kg) und Tablettierhilfsmittel K (B-i-b)) (5,94 kg) wurden mittels Saugförderer über ein 1,40 mm-Wirbelstromsieb in einen tarierten ELO-Container für rund 15 Minuten bis zur optisch erkennbaren (Sichtfenster) Homogenität vermischt. Die Herstellung der gesamten Wirbelschichtgranulat Mischung umfassend die Bestandteile B-i-a) und B-i-b) wurde in zwei Einzelansätzen in den o.g. Mengenangaben durchgeführt.
3) Herstellung der Wirbelschichtgranulate des ersten Bestandteils B-i): Diese Gesamtmenge der aus 2) erhaltenen Mischungen wurde in sechs gleiche Teile aufgeteilt und jeweils ein Wirbelschichtgranulationsprozess mit einem der sechs Teile der unter 1) hergestellten Sprühlösungen durchgeführt.
Zur Wirbelschichtgranulation wurden dabei die folgenden Parameter verwendet:

| **Parameter** | **Wert** |
|---|---|
| Anwärmen | 1min |
| Sprührate d. Sprühlsg. | 140 |
| Druck | 4 bar |
| Rütteln (s/min) | 15s |
| Zuluftklappe | 100 % |
| Zulufttemp. | 50°C |
| Abluftklappe | 25 - 40% |
| Ablufttemp. | 24 - 34°C |
| Masse aufgesprühte Sprühlsg. (kg) | 31,58 |
| Sprühzeit Sprühlsg. (min.) | 32 |
| nachgesprühtes Wasser (I) | 0,5 |
| Sprühzeit Wasser (min.) | 1 |
| Trockenzeit (min.) | 25 - 45 |
| TV (61°C / 15 min) | 1 - 1,5 % |

Feuchtemessung der erhaltenen Wirbelschichtgranulate (hier des Bestandteils B-i): Die hierin beschriebene Feuchtemessung kann unter dem Fachmann weithin bekannten Anpassungen entsprechend für die weiteren Bestandteile und Komponenten der hierin beschriebenen Schichttabletten angewendet werden): Sartorius Feuchtemessung: Die Feuchtigkeit von 2,0163 g einer erfindungsgemäßen zweiten Mischung wurde mit einem Sartorius Feuchtemesser bestimmt. Die Messung wurde durchgeführt bei 61°C mit einem Messintervall von 5 Minuten. Nach einer Messzeit von 15 Minuten betrug die Masse noch 1,9895 g, was einer Abnahme der Feuchtigkeit um ca. 1,33% entspricht. Bevorzugt ergibt sich für erfindungsgemäß erhaltene Schichttabletten aus der vorausgehend beschriebenen Feuchtemessung eine Feuchte im Bereich von 1,0 bis 1,5 %. Liegt der Wert unterhalb von < 1,0 %, lassen sich die Tabletten regelmäßig nicht homogen komprimieren und/oder neigen beim Verpressen zum Abheben der oberen Kuppel (Fachbegriff: "Deckeln"). Liegt der Wert oberhalb von 1,5 %, ist das Pressmaterial regelmäßig zu feucht und bleibt in den Pressmatrizen und an den Pressstempeln kleben, sodass sich keine stabilen Tabletten herstellen lassen.
4) Herstellung des zweiten Bestandteils B-ii): Die Rohstoffe Thiaminmononitrat (0,675 kg), Pyridoxinhydrochlorid (0,848 kg), Calciumstearat (4,42 kg), CEROGA Sodium Alginate Type NA4012 (36,20 kg) wurden abgewogen und mittels Saugförderer in einen elektrischen Mischer überführt. Die Herstellung wurde in zwei Einzelansätzen in den o.g. Mengenangaben durchgeführt.
5) Herstellung der "zweiten Mischung": Die unter 4) eingesaugten Rohstoffe wurden zusammen mit drei der unter 3) hergestellten und ebenfalls über diesen Saugförderer eingesaugten Wirbelschichtgranulate in dem o.g. elektrischen Mischer für 5 Minuten gemischt und anschließend durch ein 1,25 mm-Sieb abgelassen. Die Herstellung der "zweiten Mischung" wurde in 2 Einzelansätzen in den o.g. Mengenangaben durchgeführt.

Bezüglich der Herstellung in "Einzelansätzen" sei angemerkt, dass es sich dabei in keiner Weise um eine Beschränkung des erfindungsgemäßen Verfahrens handelt, sondern sich lediglich aufgrund der begrenzten Fassungsvermögen/Kapazitäten der jeweiligen verwendeten Geräte ergibt.

### C) Pressen

Schichttabletten wurden auf einer Doppelrundläuferpresse hergestellt.

Presswerkzeug: oblong-gerade, 22,5 mm x 9,5 mm, Bruchrille einseitig-oben;
Gewicht-Vorgaben: "Erste Mischung" A an Pressstation 1; Masse: 762,43 mg, "Zweite Mischung" B an Pressstation 2; Masse: 858,19mg, Masse Gesamt: 1620,62 mg
Vorpressen der "ersten Mischung" A: bis zu einer Härte im Bereich von 5 bis 10 N.

Anschließend wurde ein gemeinsames Verpressen der vorgepressten Mischung A und der nicht vorgepressten Mischung B zum Erhalten einer erfindungsgemäßen Schichttablette durchgeführt. Die Härteprüfung in Längsrichtung erfolgte auf einem Sollwert der Härte von mehr als 220 N.

| Parameter Tablettenpresse | Soll | Ist |
|---|---|---|
| Umdrehungen des Rotors (Moin Speed) (Rad/Min) | ca. 25 U/min | 25 |
| Filiomatic 1 ("erste Schicht" / Feeding 1) (Rad/Min) | ca. 15 U/min | 15 |
| Filiomatic 2 ("zweite Schicht" / Feeding 2) (Rad/Min) | ca. 15 U/min | 15 |
| Steghöhe - Station 1 | | 7 N |
| Steghöhe - Station 2 | | 9 N |
| Gewicht - Station 1 | | 43 N |
| Gewicht - Station 2 | | 34,32 |

Erhalten wurden dadurch Tabletten mit leichtem Glanz auf beiden Seiten der Tabletten und klarer und deutlicher Phasentrennung.

### D) Befilmen

10 kg der Tabletten wurden in einem Tabletten-Coating System (hier ACCELA-Cota®) mit Vanillin/Sepifilm-Sprühlösung gecoatet. Zur Herstellung der Sprühlösung wurde zuerst Vanillin (2,0 g) in kaltem Wasser (1918,5 g) vollständig gelöst und anschließend darin abgewogenes Sepifilm 050 Pulver (256,4 g) in einen gemäßigten Rührsog zügig, esslöffelweise dispergiert.

Nach Standzeit über Nacht wurde die Schaumschicht untergerührt und Lösung filtriert. Die Filmlösung wurde während des Sprühvorganges gerührt.

| | |
|---|---|
| Cota-Innentemperatur vor Befilmen (°C) | 10 |
| Tabletten-Vorgabe (kg) | 10 |
| Sprühdüsenentfernung v. Tablettenbett (cm) | 17,5 |
| Vorwärmen Kernbetttemperatur (°C) | 42 |
| Gesamtsprühlösungsmasse (g) | 2000 |
| eingesetzte Sprühlösung (g) | 1837 |
| Cota-Innentemperatur vor Befilmen (°C) | ca. 45 |
| Zuluftfluß (Skt.) | 12 = 440 m³ |
| Gehäuse-Unterdruck (Skt. nach links) | 0,2 |
| Zerstäubungsluft (bar) | 1,8 |
| Strahlformluft (bar) | 1,2 |
| Ablufttemperatur (°C) | ca. 37 - 33°C |
| Zuluft-Temperatur (°C) | 60 |
| Förderpumpen-Einstellung | 23 |
| Sprühgeschwindigkeit (g/min.) | 30 |
| Sprühdauer (min.) | 60 |
| Nachtrockenzeit (langsames Rotieren, Warmluft) (min.) | 5 |

Das Befilmen führte zu Schichttabletten mit glatterer Oberfläche.

### E) Freisetzungs-Untersuchungen

Zur Bestimmung der Freisetzung von Magnesium aus den Tablettenmatrices bzw. der einzelnen Schichten der erfindungsgemäßen Schichttabletten werden Leitfähigkeitsmessungen durchgeführt.

So wurde beispielsweise eine erfindungsgemäße Schichttablette, die gemäß den vorangehenden Abschnitten A) bis D) hergestellt wurde, zuerst für 5 min in 100ml entionisiertes Wasser gegeben. Während dieser Zeit löste sich die erste Schicht auf. Die verbleibende Resttablette wurde entnommen und in einer Freisetzungsapparatur analysiert.

Folgende experimentelle Parameter wurden dabei verwendet:

| | |
|---|---|
| Einwaage: | 1 Tablette, entsprechend 858 mg, |
| Messstand: | Mettler-Toledo Seven Compact Conduktivity, |
| Messelektrode: | Mettler-Toledo Inlab 731 ISM, |
| Freisetzungsapparatur: | Pharmatest PTW S 50 U/min, |
| Temperatur: | 37 °C, |
| Volumen entionisiertes Wasser: | 1000 ml, |
| Temperaturkompensation: | aus, |
| Messintervall: | 10 min. |

Dabei wurden die Werte der elektrischen Leitfähigkeit als Funktion der Zeit gemessen. Die elektrische Leitfähigkeit steigt proportional mit der Stoffmenge an gelöstem Mg²⁺ und wird daher als Maß für gelöstes Magnesium angenommen.

Da sich bei der beispielhaft untersuchten Schichttablette die erste Schicht innerhalb von 5 Minuten auflöste, die zweite Schicht aber verblieb, ist offensichtlich, dass die Mg²⁺-Ionen aus der ersten Schicht der Schichttablette gegenüber Mg²⁺-Ionen aus der zweiten Schicht bei 37 °C unter atmosphärischem Druck in Wasser schneller freigesetzt wurden.

### Härtemessung der ersten Schicht der Schichttablette

Die Messung der Härte der ersten Schicht einer erfindungsgemäßen Schichttablette erfolgte mit einem Tablettenhärte-Messgerät (SmartTest 50, SOTAX AG). Die folgenden Werte wurden bei der Messung erfindungsgemäßer erster Schichten nach Abschluss von Schritt II), einem Vorpressen der Mischung A) oder der Mischung B), um einen Schichttabletten-Vorpressling zu erhalten, gemessen.

| Parameter | Härte | Durchmesser | Breite | Dicke | Gewicht |
|---|---|---|---|---|---|
| | [N] | [mm] | [mm] | [mm] | [mg] |
| 1 | 6 | 33,32 | 9,36 | 6,62 | 780,6 |
| 2 | 6 | 22,30 | 9,40 | 6,62 | 773,5 |
| 3 | 6 | 22,31 | 9,45 | 6,67 | 786,6 |
| 4 | 7 | 22,30 | 9,41 | 6,69 | 777,0 |
| 5 | 6 | 22,29 | 9,37 | 6,63 | 779,6 |
| 6 | 6 | 22,31 | 9,38 | 6,59 | 774,9 |
| 7 | 9 | 22,31 | 9,41 | 6,52 | 773,3 |
| 8 | 7 | 22,32 | 9,52 | 6,70 | 798,7 |
| 9 | 7 | 22,31 | 9,43 | 6,49 | 767,5 |
| 10 | 7 | 22,32 | 9,38 | 6,52 | 771,3 |

Statistisch ergeben sich durch Wertung der vorausgehend genannten Einzelmessungen die folgenden Werte:

| Parameter | Härte | Durchmesser | Breite | Dicke | Gewicht |
|---|---|---|---|---|---|
| Einheit | [N] | [mm] | [mm] | [mm] | [mg] |
| Anzahl Werte | 10 | 10 | 10 | 10 | 10 |
| Mittelwert | 7 | 22,31 | 9,41 | 6,61 | 778,3 |
| Minimum | 9 | 22,32 | 9,52 | 6,70 | 798,7 |
| Maximum | 6 | 22,29 | 9,36 | 6,49 | 767,5 |
| Relative Standardabweichung | 14,2% | 0,045% | 0,502% | 1,122% | 1,15% |

### Härtemessung der Schichttablette

Die Messung der erfindungsgemäßen Schichttablette erfolgte wie vorausgehend (siehe Härtemessung der ersten Schicht der Schichttablette) beschrieben entsprechend für erfindungsgemäße Schichttabletten. Die folgenden Werte wurden bei der Messung erfindungsgemäßer Schichtttablette gemessen.

| Parameter | Härte | Durchmesser | Breite | Dicke | Gewicht |
|---|---|---|---|---|---|
| Einheit | [N] | [mm] | [mm] | [mm] | [mg] |
| 1 | 248 | 22,36 | 9,23 | 814 | 1612,1 |
| 2 | 250 | 22,37 | 9,25 | 799 | 1598,0 |
| 3 | 263 | 22,37 | 9,33 | 808 | 1613,4 |
| 4 | 244 | 22,39 | 9,18 | 798 | 1592,7 |
| 5 | 242 | 22,38 | 9,18 | 803 | 1596,7 |
| 6 | 247 | 22,38 | 9,19 | 808 | 1614,5 |
| 7 | 227 | 22,37 | 9,21 | 814 | 1601,7 |
| 8 | 230 | 22,39 | 9,18 | 808 | 1608,9 |
| 9 | 241 | 22,36 | 9,25 | 801 | 1597,6 |
| 10 | 227 | 22,38 | 9,22 | 807 | 16,093 |

Statistisch ergeben sich durch Wertung der vorausgehend genannten Einzelmessungen die folgenden Werte:

| Parameter | Härte | Durchmesser | Breite | Dicke | Gewicht |
|---|---|---|---|---|---|
| Einheit | [N] | [mm] | [mm] | [mm] | [mg] |
| Anzahl Werte | 10 | 10 | 10 | 10 | 10 |
| Mittelwert | 200 | 22,50 | 780 | 950 | 1591,2 |
| Minimum | 242 | 22,38 | 922 | 806 | 1604,5 |
| Maximum | 263 | 22,39 | 933 | 814 | 1614,5 |
| Relative Standardabweichung | 4,7% | 0,048% | 0,508% | 0,702% | 0,50% |

## Patentansprüche

1. Verfahren zur Herstellung einer Schichttablette umfassend oder bestehend aus einer ersten, Mg²⁺-Ionen freisetzenden, Schicht und einer zweiten, Mg²⁺-Ionen freisetzenden, Schicht umfassend oder bestehend aus den Schritten
I) Bereitstellen
A) einer ersten Mischung zur Herstellung der ersten Schicht, wobei die erste Mischung die folgenden Bestandteile enthält oder aus den folgenden Bestandteilen besteht:
A-i) einem oder mehreren Magnesium-Salzen ausgewählt aus der Gruppe, bestehend aus Magnesiumoxid, Magnesiumhydroxid, Magnesiumcarbonat, Trimagnesiumdicitrat, Magnesiumgluconat, Magnesiummalat, Magnesiumglycinat, Magnesiumorotat, Magnesiumacetat, Magnesiumascorbat, Magnesiumformiat, Magnesiumfumarat, Magnesiumglutamat, Magnesiumglycerophosphat, Magnesiumlactat, Magnesiumnitrat, Magnesiumoxalat, Magnesiumphosphat, Magnesiumpidolat, Magnesiumpropionat, Magnesiumtartrat, Magnesiumaspartat, Magnesiumchlorid und Magnesiumsulfat,
A-ii) einem oder mehreren Vitaminen, vorzugsweise B-Vitaminen,
A-iii) einem oder mehreren Sprengmitteln,
A-iv) einem oder mehreren Trägerstoffen,
und optional
A-v) einem oder mehreren Pigmenten oder Farbstoffen,
und optional
A-vi) einem oder mehreren Hilfsstoffen, vorzugsweise weiteren Hilfsstoffen
und
B) einer zweiten Mischung zur Herstellung der zweiten Schicht, wobei die zweite Mischung die folgenden Bestandteile enthält oder aus den folgenden Bestandteilen besteht:
B-i) mindestens einer ersten Komponente, umfassend oder bestehend aus:
B-i-a) einem oder mehreren Magnesium-Salzen ausgewählt aus der Gruppe, bestehend aus Magnesiumoxid, Magnesiumhydroxid, Magnesiumcarbonat, Trimagnesiumdicitrat, Magnesiumgluconat, Magnesiummalat, Magnesiumglycinat, Magnesiumorotat, Magnesiumacetat, Magnesiumascorbat, Magnesiumformiat, Magnesiumfumarat, Magnesiumglutamat, Magnesiumglycerophosphat, Magnesiumlactat, Magnesiumnitrat, Magnesiumoxalat, Magnesiumphosphat, Magnesiumpidolat, Magnesiumpropionat, Magnesiumtartrat, Magnesiumaspartat, Magnesiumchlorid und Magnesiumsulfat,
B-i-b) einem oder mehreren Tablettierhilfsmitteln,
und
B-i-c) einem oder mehreren Trägerstoffen zur Feuchtgranulierung
und
B-ii) mindestens einer zweiten Komponente, umfassend oder bestehend aus:
B-ii-a) einem oder mehreren Vitaminen, vorzugsweise B-Vitaminen,
und optional
B-ii-b) einem oder mehreren Hilfsstoffen, vorzugsweise weiteren Hilfsstoffen
wobei die bzw. mindestens eine Komponente der Zusammensetzung B-i) durch ein Verfahren zur Feuchtgranulierung, vorzugsweise der Wirbelschichtgranulation, hergestellt ist,
und
wobei B-i) und B-ii) miteinander gemischt sind,
wobei die Mischungen A) und B) unabhängig voneinander jeweils als Granulat oder Pulver, vorzugsweise als Feuchtgranulat, bereitgestellt werden,
II) Vorpressen der Mischung A) oder der Mischung B), um einen Schichttabletten-Vorpressling zu erhalten,
III) Hinzufügen der nicht vorgepressten Mischung A) oder B),
IV) gemeinsames Verpressen der Mischungen A) und B) zu einer Schichttablette und optional
V) Befilmen der Schichttablette, um einen Film C) auf der Oberfläche der Schichttablette zu erhalten,
**dadurch gekennzeichnet, dass** Mg²⁺-Ionen aus der ersten Schicht gegenüber Mg²⁺-Ionen aus der zweiten Schicht bei Körpertemperatur unter atmosphärischem Druck in Wasser schneller freigesetzt werden.

2. Verfahren zur Herstellung einer Schichttablette gemäß Anspruch 1, wobei die erste Mischung zur Herstellung der ersten Schicht die folgenden Bestandteile enthält oder aus den folgenden Bestandteilen besteht:
A-i) Magnesiumhydroxid und/oder Magnesiumcarbonat,
A-ii) Cyanocobalamin und/oder Riboflavin,
A-iii) Magnesiumcarbonat,
A-iv) quervernetzte Natrium Carboxymethylcellulose, amorphe hochdisperse Kieselsäure,
A-v) Eisenoxid,
und
A-vi) Calciumstearat,
und/oder
wobei die zweite Mischung zur Herstellung der zweiten Schicht die folgenden Bestandteile enthält oder aus den folgenden Bestandteilen besteht:
B-i) mindestens einer ersten Komponente, umfassend oder bestehend aus:
B-i-a) Trimagnesiumdicitrat 9-Hydrat,
B-i-b) Cellulosepulver,
und
B-i-c) Trägerstoffe zur Feuchtgranulierung ausgewählt aus der Gruppe, bestehend aus Alginaten, Hydroxypropylmethylcellulose, und Sorbit enthaltendes Pulver
und
B-ii) mindestens einer zweiten Komponente, umfassend oder bestehend aus:
B-ii-a) Thiaminmononitrat und/oder Pyridoxinhydrochlorid,
und optional
B-ii-b) Calciumstearat.

3. Verfahren zur Herstellung einer Schichttablette gemäß einem der vorausgehenden Ansprüche wobei
die erste Mischung A) in Schritt II) vorgepresst wird,
und/oder
Schritt II) bis zum Erreichen einer Härte der Mischung A) oder B) in mindestens einer räumlichen Richtung des Schichttabletten-Vorpresslings im Bereich von 5 - 15 N, vorzugsweise im Bereich von 6 - 13 N, besonders bevorzugt im Bereich von 7 - 11 N durchgeführt wird,
und/oder
Schritt IV) bis zum Erreichen einer Härte der Schichttablette in mindestens einer räumlichen Richtung der Schichttablette, vorzugsweise, falls vorhanden, in Längsrichtung der Schichttablette, im Bereich von 200 - 250 N, vorzugsweise im Bereich von 210 - 240 N, besonders bevorzugt im Bereich von 220 - 230 N durchgeführt wird.

4. Verfahren zur Herstellung einer Schichttablette gemäß einem der vorausgehenden Ansprüche, wobei Schritt II) und/oder Schritt IV) das Erzeugen mindestens einer oder mehrerer Bruchrillen auf mindestens einer Seite des Schichttabletten-Vorpresslings oder der Schichttablette umfasst
oder
umfassend mindestens einen zusätzlichen Schritt des Erzeugens mindestens einer oder mehrerer Bruchrillen auf mindestens einer Seite des Schichttabletten-Vorpresslings oder der Schichttablette.

5. Verfahren zur Herstellung einer Schichttablette gemäß einem der vorausgehenden Ansprüche, wobei
das Gewichtsverhältnis der ersten Mischung A) zur zweiten Mischung B) im Bereich von 1 : 3 bis 3 : 1 vorzugsweise im Bereich von 1 : 1,5 bis 1,5 : 1, besonders bevorzugt im Bereich von 1 : 1,1 bis 1,1 : 1 liegt
und/oder
das Gesamtgewicht der Schichttablette im Bereich von 10 mg bis 2500 mg, vorzugsweise im Bereich von 700 mg bis 2000 mg, besonders bevorzugt im Bereich von 1400 mg bis 1800 mg liegt,
und/oder
das Gewichtsverhältnis des Films C), falls vorhanden, zur Summe aus der ersten Mischung A) und der zweiten Mischung B) im Bereich von 1 : 80 bis 1 : 10, vorzugsweise im Bereich von 1 : 40 bis 1 : 25, besonders bevorzugt im Bereich von 1 : 35 bis 1 : 30 liegt,
und/oder
die Schichttablette einen Gehalt an Magnesium bzw. Mg²⁺-Ionen im Bereich von 100 mg bis 1000 mg, vorzugsweise im Bereich von 200 mg bis 750 mg, besonders bevorzugt im Bereich von 250 mg bis 500 mg aufweist,
und/oder
das Gewichtsverhältnis des durch die erste Mischung A) bereitgestellten Gehalts an Magnesium bzw. Mg²⁺-Ionen zu dem durch die zweite Mischung B) bereitgestellten Gehalt an Magnesium bzw. Mg²⁺-Ionen im Bereich von 1 : 6 bis 6 : 1, vorzugsweise im Bereich von 1 : 4 bis 4 : 1, besonders bevorzugt im Bereich von 1 : 3 bis 3 : 1 liegt.

6. Verfahren zur Herstellung einer Schichttablette gemäß einem der vorausgehenden Ansprüche, wobei
die erste Mischung A) mindestens einen Hilfsstoff, vorzugsweise weiteren Hilfsstoff, enthält und/oder die zweite Mischung B) unabhängig davon mindestens einen Hilfsstoff, vorzugsweise weiteren Hilfsstoff, enthält, wobei der mindestens eine Hilfsstoff bzw. die Hilfsstoffe, unabhängig voneinander, ausgewählt ist bzw. sind aus der Gruppe, bestehend aus Süßungsmitteln, vorzugsweise Laktose, Saccharose oder Sorbit; Säureregulatoren, vorzugsweise Kaliumtartrat; Trennmitteln, vorzugsweise Salzen, insbesondere Calciumsalzen, von Speisefettsäuren, vernetzter Natriumcarboxymethylcellulose oder Siliciumdioxid; Säuerungsmitteln, vorzugsweise Zitronensäure; Aromen, vorzugsweise natürlichen Aromen; Füllstoffen, vorzugsweise Hydroxypropylcellulose; weiteren Citratsalzen, vorzugsweise Natriumcitrat, Kaliumcitrat, Kalium-Natrium-Hydrogencitrat, Calcium-Natrium-Hydrogencitrat, Kupfercitrat und/oder Eisencitrat; Calciumcarbonat, Calciumlactat, Natriumhydrogencarbonat; L-Carnitin; Maltodextrin; Molybdän, vorzugsweise Natriummolybdat; Chrom, vorzugsweise Chromchlorid; und Selen, vorzugsweise Natriumselenit, Natriumselenat oder Selenhefe; vorzugsweise wobei der mindestens eine Hilfsstoff ausgewählt ist aus der Gruppe, bestehend aus mikrokristalliner Cellulose, Hydroxypropylcellulose, Sorbit, Stearinsäure und Alginat
und/oder
die erste Mischung A) und/oder die zweite Mischung B) einen oder mehrere Farbstoffe und/oder Pigmente in einer Menge enthält bzw. enthalten, sodass die erste Schicht von der zweiten Schicht der Schichttablette visuell durch das menschliche Auge, vorzugsweise auch bei Farb-Fehlsichtigkeiten bzw. nicht vollständig funktionsfähiger Polychromasie, unterschieden werden kann.

7. Verfahren zur Herstellung einer Schichttablette gemäß einem der vorausgehenden Ansprüche, wobei der Film C) besteht aus oder enthält:
- eine, mehrere oder sämtliche Verbindungen ausgewählt aus der Gruppe, bestehend aus Hydroxypropylmethylcellulosen oder Hypromellose, Polyethylene Glycol, Talkum, mikrokristallinen Cellulosen und acetylierten Monoglyceriden,
und optional
- einen Geschmacksstoff.

8. Verfahren zur Herstellung einer Schichttablette gemäß einem der vorausgehenden Ansprüche, wobei die in Schritt I) bereitgestellten Mischungen A) und/oder B) unabhängig voneinander eine Feuchte im Bereich von 0,1 bis 10 %, vorzugsweise im Bereich von 0,5 bis 5 %, besonders bevorzugt im Bereich von 1 bis 2 %, bezogen auf das Gesamtgewicht der jeweiligen Mischung A) oder B), aufweisen.

9. Verfahren zur Herstellung einer Schichttablette gemäß einem der vorausgehenden Ansprüche, wobei in der Wirbelschichtgranulation als Verfahren zur Feuchtgranulierung, falls verwendet, die Bestandteile der einen, mehreren oder sämtlichen Komponenten B-ii) auf die Bestandteile der einen bzw. mehreren optional bereits beschichteten Komponenten B-i) einer, mehrere oder sämtliche der folgenden Parameter verwendet werden:
- Zulufttemperatur im Bereich von 20 bis 80 °C, vorzugsweise im Bereich von 25 bis 60 °C besonders bevorzugt im Bereich von 30 bis 40 °C,
- Sprühluftdruck im Bereich von 1 bis 7 bar, vorzugsweise im Bereich von 1 bis 4 bar, besonders bevorzugt im Bereich von 1,25 bis 1,75 bar,
- Rüttelzeit im Bereich von 5 bis 25 s, vorzugsweise im Bereich von 6 bis 19 s, besonders bevorzugt im Bereich von 7 bis 17 s.

10. Schichttablette erhalten durch oder erhältlich gemäß einem Verfahren nach einem der vorausgehenden Ansprüche.

11. Schichttablette gemäß Anspruch10, wobei der Film C), falls vorhanden, die Schichttablette weitestgehend vollständig, vorzugsweise vollständig, besonders bevorzugt vollständig und gleichmäßig bedeckt.

12. Schichttablette gemäß einem der Ansprüche 10 oder 11, wobei die Schichttablette frei von einem, mehreren oder sämtlichen der Bestandteile ausgewählt aus der Gruppe bestehend aus Laktose, Iod, Gluten, Aromastoffen und Süßungsmitteln ist, vorzugsweise frei ist von Laktose, Iod und Gluten ist.

13. Schichttablette gemäß einem der Ansprüche 10 bis 12 zur Anwendung als Medikament, vorzugsweise zur Anwendung bei der Behandlung und/oder Vorbeugung einer Stoffwechselerkrankung und/oder Mineralstoffmangelerkrankung, wobei die Stoffwechselerkrankung und/oder Mineralstoffmangelerkrankung vorzugsweise ausgewählt ist aus der Gruppe, bestehend aus Hypomagnesiämie, Herzrhythmusstörungen, Migräne und Hypertonie.

14. Schichttablette zur Anwendung gemäß Anspruch 13, wobei mindestens eine unerwünschte Nebenwirkung durch die durch die Schichttablette bedingte erhöhte Aufnahme von Magnesium in den menschlichen Körper hinsichtlich Stärke der mindestens einen Nebenwirkung und/oder Häufigkeit der mindestens einen Nebenwirkung verringert sind oder nicht auftreten, wobei die mindestens eine Nebenwirkung ausgewählt ist bzw. sind aus der Gruppe, bestehend aus abführender Wirkung, weicher Stuhl, Durchfall und Müdigkeitserscheinungen.

## Claims

1. A method for manufacturing a layered tablet comprising or consisting of a first, Mg²⁺ ion-releasing, layer and a second, Mg²⁺ ion-releasing, layer comprising or consisting of the steps
I) providing
A) a first mixture for producing the first layer, wherein the first mixture contains or consists of the following components
A-i) one or more magnesium salts selected from the group consisting of magnesium oxide, magnesium hydroxide, magnesium carbonate, trimagnesium dicitrate, magnesium gluconate, magnesium malate, magnesium glycinate, magnesium orotate, magnesium acetate, magnesium ascorbate, magnesium formate, magnesium fumarate, magnesium glutamate, magnesium glycerophosphate, magnesium lactate, magnesium nitrate, magnesium oxalate, magnesium phosphate, Magnesium pidolate, magnesium propionate, magnesium tartrate, magnesium aspartate,Magnesium chloride and magnesium sulfate,
A-ii) one or more vitamins, preferably B vitamins,
A-iii) one or more disintegrating agents,
A-iv) one or more carrier materials,
and optionally
A-v) one or more pigments or colorants,
and optionally
A-vi) one or more excipients, preferably further excipients
and
B) a second mixture for manufacturing the second layer, wherein the second layer comprises or consists of the following components:
B-i) at least one first component, comprising or consisting of:
B-i-a) one or more magnesium salts selected from the group consisting of magnesium oxide, magnesium hydroxide, magnesium carbonate, trimagnesium dicitrate, magnesium gluconate, magnesium malate, magnesium glycinate, magnesium orotate, magnesium acetate, magnesium ascorbate, magnesium formate, magnesium fumarate, magnesium glutamate, magnesium glycerophosphate, magnesium lactate, magnesium nitrate, magnesium oxalate, magnesium phosphate, Magnesium pidolate, magnesium propionate, magnesium tartrate, magnesium aspartate,Magnesium chloride and magnesium sulfate,
B-i-b) one or more tabletting aids,
and
B-i-c) one or more carrier materials for wet granulation,
and
B-ii) at least one second component, comprising or consisting of:
B-ii-a) one or more vitamins, preferably B vitamins,
and optionally
B-ii-b) one or more excipients, preferably further excipients
wherein the or at least one of the components of composition B-i) is manufactured by a process of wet granulation, preferably of fluidized bed granulation,
and
wherein B-i) and B-ii) are mixed,
wherein the mixtures A) and B) are provided independently from each other as granulate or powder, preferably as wet granulate,
II) pre-pressing of the mixture A) or mixture B), to yield a pre-pressed layered tablet,
III) adding the non pre-pressed mixture A) or B),
IV) combined pressing of the mixtures A) and B) for a layered tablet
and optionally
V) coating of the layered tablet, to yield a film C) at the surface of the layered tablet,
**characterized in that** Mg²⁺ ions are released faster from the first layer compared to Mg²⁺ ions from the second layer in water, at body temperature and atmospheric pressure.

2. The method for manufacturing a layered tablet according to claim 1, wherein the first mixture for manufacturing of the first layer comprises or consists of the following components:
A-i) magnesium hydroxide and/or magnesium carbonate
A-ii) cyanocobalmin and/or riboflavin
A-iii) magnesium carbonate
A-iv) crosslinked sodium carboxymethylcellulose, amorphous highly dispersed silicic acid,
A-v) iron oxide an
d
A-vi) calcium stearate,
and/or
wherein the second mixture for manufacturing the second layer consists of or comprises the following components:
B-i) at least one first component, comprising or consisting of:
B-i-a) trimagnesiumdicitrate 9-hydrate,
B-i-b) powdered cellulose
and
B-i-c) carrier materials for wet granulation selected from the group consisting of alginates, hydroxypropyl methylcellulose, and sorbit containing powder
and
B-ii) at least one second component, comprising or consisting of
B-ii-a) thiaminmononitrat and/or pyidoxinhydrochloride
and optionally
B-ii-b) calciumstearat.

3. The method for manufacturing a layered tablet according to one of the preceding claims, wherein
the first mixture A) is pre-pressed in step II),
and/or
step II) is carried out until a hardness of mixture A) or B) is reached in at least one spatial direction of the layered tablet pre-pressed in the range of 5 - 15 N, preferably in the range of 6 - 13 N, particularly preferably in the range of 7 - 11 N,
and/or
step IV) is carried out until the hardness of the layered tablet is reached in at least one spatial direction of the layered tablet, preferably, if present, in the longitudinal direction of the layered tablet, in the range of 200 - 250 N, preferably in the range of 210 - 240 N, particularly preferably in the range of 220 - 230 N.

4. The method for producing a layered tablet according to one of the preceding claims, wherein step II) and/or step IV) comprises creating at least one or more fracture grooves on at least one side of the layered tablet preform or the layered tablet
or
comprising at least one additional step of creating at least one or more fracture grooves on at least one side of the layered tablet preform or tablet.

5. The method for manufacturing a layered tablet according to one of the preceding claims, wherein
the weight ratio of the first mixture A) to the second mixture B) is in the range of 1:3 to 3:1, preferably in the range of 1:1.5 to 1.5:1, particularly preferably in the range of 1:1.1 to 1.1:1
and/or
the total weight of the layered tablet is in the range of 10 mg to 2500 mg, preferably in the range of 700 mg to 2000 mg, particularly preferably in the range of 1400 mg to 1800 mg,
and/or
the weight ratio of the film C), if present, to the sum of the first mixture A) and the second mixture B) is in the range of 1 : 80 to 1 : 10, preferably in the range of 1 : 40 to 1 : 25, especially preferably in the range of 1 : 35 to 1 : 30,
and/or
the layered tablet has a magnesium or Mg²⁺ ion content in the range of 100 mg to 1000 mg, preferably in the range of 200 mg to 750 mg, particularly preferably in the range of 250 mg to 500 mg,
and/or
the weight ratio of the content of magnesium or Mg²⁺-ions provided by the first mixture A) to the content of magnesium or Mg²⁺-ions provided by the second mixture B) is in the range from 1:6 to 6:1, preferably in the range from 1:4 to 4:1, particularly preferably in the range from 1:3 to 3:1.

6. The method for manufacturing a layered tablet according to one of the preceding claims, wherein
the first mixture A) contains at least one excipient, preferably another excipient and/or the second mixture B) independently thereof contains at least one excipient, preferably a further excipient, wherein the at least one excipient or excipients, independently of one another, is/are selected from the group consisting of sweeteners, preferably lactose, sucrose or sorbitol; acidity regulators, preferably potassium tartrate; separating agents, preferably salts, in particular calcium salts, of fatty acids, crosslinked sodium carboxymethylcellulose or silicon dioxide; acidifying agents, preferably citric acid; flavours, preferably natural flavours; fillers, preferably hydroxypropyl cellulose; other citrate salts, preferably sodium citrate, potassium citrate, potassium-sodium hydrogen citrate, calcium-sodium hydrogen citrate, copper citrate and/or iron citrate; calcium carbonate, calcium lactate, sodium hydrogen carbonate; L-carnitine; maltodextrin; molybdenum, preferably sodium molybdate; chromium, preferably chromium chloride; and selenium, preferably sodium selenite, sodium selenate or selenium yeast; preferably wherein the at least one excipient is selected from the group consisting of microcrystalline cellulose, hydroxypropyl cellulose, sorbitol, stearic acid and alginate
and/or
the first mixture A) and/or the second mixture B) contain one or more colorants and/or pigments in an amount such that the first layer of the layered tablet can be visually distinguished from the second layer of the layered tablet by the human eye, preferably also for colour vision defects or not fully functional polychromasia.

7. The method for producing a layered tablet according to one of the preceding claims, wherein the film C) consists of or contains
- one, several or all compounds selected from the group consisting of hydroxypropylmethylcelluloses or hypromellose, polyethylene glycol, talc, microcrystalline celluloses and acetylated monoglycerides, and
- optionally a flavouring agent.

8. The method for producing a layered tablet according to one of the preceding claims, wherein the mixtures A) and/or B) provided in step I) independently of one another have a moisture content in the range from 0.1 to 10 %, preferably in the range from 0.5 to 5 %, particularly preferably in the range from 1 to 2 %, based on the total weight of the respective mixture A) or B).

9. The method for producing a layered tablet according to one of the preceding claims, wherein in the fluidized bed granulation as a wet granulation process, if used, the components of the one, several or all of the components B-ii) are applied to the components of the one or more optionally already coated components B-i) one, several or all of the following parameters are used:
- supply air temperature in the range of 20 to 80 °C, preferably in the range of 25 to 60 °C, particularly preferably in the range of 30 to 40 °C,
- spray air pressure in the range of 1 to 7 bar, preferably in the range of 1 to 4 bar, particularly preferably in the range of 1.25 to 1.75 bar,
- vibration time in the range of 5 to 25 s, preferably in the range of 6 to 19 s, particularly preferably in the range of 7 to 17 s.

10. A layered tablet obtained by or obtainable by a process according to one of the preceding claims.

11. The layered tablet according to claim 10, wherein the film C), if present, covers the layered tablet as far as possible completely, preferably completely, particularly preferably completely and uniformly.

12. The layered tablet according to one of the claims 10 or 11, wherein the layered tablet is free of one, several or all of the ingredients selected from the group consisting of lactose, iodine, gluten, flavourings and sweeteners, preferably is free of lactose, iodine and gluten.

13. The layered tablet according to one of claims 10 to 12 for use as a medicament, preferably for use in the treatment and/or prevention of a metabolic disease and/or mineral deficiency disease, wherein the metabolic disease and/or mineral deficiency disease is preferably selected from the group consisting of hypomagnesiamia, cardiac arrhythmia, migraines and hypertension.

14. The layered tablet for use according to claim 13, wherein at least one undesirable side effect is reduced or does not occur due to the increased intake of magnesium into the human body caused by the layered tablet with respect to the strength of the at least one side effect and/or frequency of the at least one side effect, wherein the at least one side effect is/are selected from the group consisting of laxative effect, soft stool, diarrhoea and fatigue symptoms.

## Revendications

1. Procédé de production d'un comprimé stratifié comprenant ou se composant d'une première couche qui libère des ions Mg²⁺ et d'une deuxième couche qui libère des ions Mg²⁺, comprenant ou consistant en les étapes
I) fournir
A) un premier mélange pour produire la première couche, dans lequel le premier mélange contient les ingrédients suivants ou est constitué des ingrédients suivants:
A-i) un ou plusieurs sels de magnésium choisis dans le groupe constitué par l'oxyde de magnésium, l'hydroxyde de magnésium, le carbonate de magnésium, le dicitrate de trimagnésium, le gluconate de magnésium, le malate de magnésium, le glycinate de magnésium, l'orotate de magnésium, l'acétate de magnésium, l'ascorbate de magnésium, le formiate de magnésium, le fumarate de magnésium, le glutamate de magnésium, le glycérophosphate de magnésium, le lactate de magnésium, le nitrate de magnésium, l'oxalate de magnésium, le phosphate de magnésium, le pidolate de magnésium, le propionate de magnésium, le tartrate de magnésium, l'aspartate de magnésium, le chlorure de magnésium et le sulfate de magnésium,
A-ii) une ou plusieurs vitamines, de préférence des vitamines B,
A-iii) un ou plusieurs désintégrants,
A-iv) un ou plusieurs supports,
et en option
A-v) un ou plusieurs pigments ou colorants,
et en option
A-vi) un ou plusieurs excipients, de préférence d'autres excipients
et
B) un deuxième mélange pour produire la deuxième couche, dans lequel le deuxième mélange contient les ingrédients suivants ou est constitué des ingrédients suivants:
B-i) au moins un premier ingrédient comprenant ou consistant en:
B-i-a) un ou plusieurs sels de magnésium choisis dans le groupe constitué par l'oxyde de magnésium, l'hydroxyde de magnésium, le carbonate de magnésium, le dicitrate de trimagnésium, le gluconate de magnésium, le malate de magnésium, le glycinate de magnésium, l'orotate de magnésium, l'acétate de magnésium, l'ascorbate de magnésium, le formiate de magnésium, le fumarate de magnésium, le glutamate de magnésium, le glycérophosphate de magnésium, le lactate de magnésium, le nitrate de magnésium, l'oxalate de magnésium, le phosphate de magnésium, le pidolate de magnésium, le propionate de magnésium, le tartrate de magnésium, l'aspartate de magnésium, le chlorure de magnésium et le sulfate de magnésium,
B-i-b) un ou plusieurs excipients de mise en forme de comprimé,
et
B-i-c) un ou plusieurs supports pour la granulation humide
et
B-ii) au moins un deuxième composant comprenant ou consistant en:
B-ii-a) une ou plusieurs vitamines, de préférence des vitamines B,
et en option
B-ii-b) un ou plusieurs excipients, de préférence d'autres excipients
dans lequel ledit ou bien au moins un composant de la composition B-i) est produit par un procédé de granulation humide, de préférence de la granulation en lit fluidisé,
et
dans lequel B-i) et B-ii) sont mélangés l'un avec l'autre,
dans lequel les mélanges A) et B) sont chacun fournis indépendamment l'un de l'autre en tant que granulés ou poudre, de préférence en tant que granulés humides,
II) pré-comprimer le mélange A) ou le mélange B) afin d'obtenir un pré-comprimé de comprimé stratifié,
III) ajouter le mélange A) ou B) non pré-comprimé,
IV) comprimer ensemble les mélanges A) et B) pour obtenir un comprimé stratifié et en option
V) pelliculer le comprimé stratifié pour obtenir un film C) à la surface du comprimé stratifié,
**caractérisé par le fait que** des ions Mg²⁺ de la première couche sont libérés plus rapidement dans l'eau, à température corporelle sous pression atmosphérique, que des ions Mg²⁺- de la deuxième couche.

2. Procédé de production d'un comprimé stratifié selon la revendication 1, dans lequel le premier mélange destiné à produire la première couche contient les ou est constitué des ingrédients suivants:
A-i) l'hydroxyde de magnésium et/ou le carbonate de magnésium,
A-ii) la cyanocobalamine et/ou la riboflavine,
A-iii) le carbonate de magnésium,
A-iv) la carboxyméthylcellulose de sodium réticulée, la silice amorphe hautement dispersée,
A-v) l'oxyde de fer,
et
A-vi) le stéarate de calcium,
et/ou
dans lequel le deuxième mélange destiné à produire la deuxième couche contient les ou est constitué des ingrédients suivants:
B-i) au moins un premier composant comprenant ou consistant en:
B-i-a) le dicitrate de trimagnésium nonahydraté,
B-i-b) la poudre de cellulose,,
et
B-i-c) des supports pour la granulation humide choisis dans le groupe constitué par des alginates, de l'hydroxypropylméthylcellulose et de la poudre contenant du sorbitol
et
B-ii) au moins un deuxième composant comprenant ou consistant en:
B-ii-a) le mononitrate de thiamine et/ou le chlorhydrate de pyridoxine,
et en option
B-ii-b) le stéarate de calcium.

3. Procédé de production d'un comprimé stratifié selon l'une quelconque des revendications précédentes, dans lequel
le premier mélange A) est pré-comprimé à l'étape II),
et/ou
l'étape II) est mise en œuvre jusqu'à ce que le mélange A) ou B) ait atteint une dureté, dans au moins une direction spatiale du pré-comprimé de comprimé stratifié, qui se situe dans la plage allant de 5 à 15 N, de préférence dans la plage allant de 6 à 13 N, de manière particulièrement préférée dans la plage allant de 7 à 11 N,
et/ou
l'étape IV) est mise en œuvre jusqu'à ce que le comprimé stratifié ait atteint une dureté, dans au moins une direction spatiale du comprimé stratifié, de préférence, si elle est présente, dans la direction longitudinale du comprimé stratifié, qui se situe dans la plage allant de 200 à 250 N, de préférence dans la plage allant de 210 à 240 N, de manière particulièrement préférée dans la plage allant de 220 à 230 N.

4. Procédé de production d'un comprimé stratifié selon l'une quelconque des revendications précédentes, dans lequel l'étape II) et/ou l'étape IV) comprend la génération d'au moins une ou de plusieurs barres de cassure sur au moins une face du pré-comprimé de comprimé stratifié ou du comprimé stratifié
ou
comprenant au moins une étape supplémentaire de génération d'au moins une ou de plusieurs barres de cassure sur au moins une face du pré-comprimé de comprimé stratifié ou du comprimé stratifié.

5. Procédé de production d'un comprimé stratifié selon l'une quelconque des revendications précédentes, dans lequel
le rapport pondéral du premier mélange A) au deuxième mélange B) se situe dans la plage allant de 1 : 3 à 3 : 1, de préférence dans la plage allant de 1 : 1,5 à 1,5 : 1, de manière particulièrement préférée dans la plage allant de 1 : 1,1 à 1,1 : 1
et/ou
le poids total du comprimé stratifié est compris entre 10 mg et 2500 mg, de préférence entre 700 mg et 2000 mg, de manière particulièrement préférée entre 1400 mg et 1800 mg,
et/ou
le rapport pondéral du film C), s'il est présent, à la somme du premier mélange A) et du deuxième mélange B) se situe dans la plage allant de 1 : 80 à 1 :10, de préférence dans la plage allant de 1 : 40 à 1 : 25, de manière particulièrement préférée dans la plage allant de 1 : 35 à 1 : 30,
et/ou
le comprimé stratifié présente une teneur en magnésium ou bien en ions Mg²⁺ qui est comprise entre 100 mg et 1000 mg, de préférence comprise entre 200 mg et 750 mg, de manière particulièrement préférée comprise entre 250 mg et 500 mg, et/ou
le rapport pondéral de la teneur en magnésium ou bien en ions Mg²⁺ fournie par le premier mélange A) à la teneur en magnésium ou bien en ions Mg²⁺ fournie par le deuxième mélange B) se situe dans la plage allant de 1 : 6 à 6 : 1, de préférence dans la plage allant de 1 : 4 à 4 : 1, de manière particulièrement préférée dans la plage allant de 1 : 3 à 3 : 1.

6. Procédé de production d'un comprimé stratifié selon l'une quelconque des revendications précédentes, dans lequel
le premier mélange A) contient au moins un excipient, de préférence un autre excipient, et/ou le deuxième mélange B) contient, indépendamment de ceci, au moins un excipient, de préférence un autre excipient, ledit au moins un excipient ou bien les excipients étant choisi(s), indépendantes les uns des autres, dans le groupe constitué par les édulcorants, de préférence le lactose, le saccharose ou le sorbitol; les correcteurs d'acidité, de préférence le tartrate de potassium; les anti-agglomérants, de préférence des sels, en particulier les sels de calcium, d'acides gras, de carboxyméthylcellulose sodique réticulée ou de dioxyde de silicium; les acidifiants, de préférence l'acide citrique; les arômes, de préférence les arômes naturels; les charges, de préférence l'hydroxypropylcellulose; d'autres sels de citrate, de préférence le citrate de sodium, le citrate de potassium, le citrate d'hydrogène de potassium et de sodium, le citrate d'hydrogène de sodium et de calcium, le citrate de cuivre et/ou le citrate de fer; le carbonate de calcium, le lactate de calcium, l'hydrogénocarbonate de sodium, la L-carnitine; la maltodextrine; le molybdène, de préférence le molybdate de sodium; le chrome, de préférence le chlorure de chrome; et le sélénium, de préférence le sélénite de sodium, le sélénate de sodium ou la levure de sélénium; de préférence dans lequel ledit au moins un excipient est choisi dans le groupe constitué par la cellulose microcristalline, l'hydroxypropylcellulose, le sorbitol, l'acide stéarique et l'alginate
et/ou
le premier mélange A) et/ou le deuxième mélange B) contient ou bien contiennent un ou plusieurs colorants et/ou pigments en une quantité telle que l'on peut distinguer de manière visuelle à l'œil humain la première couche de la deuxième couche du comprimé stratifié, de préférence également dans le cas de dyschromatopsie ou bien d'un polychromatisme qui n'est pas entièrement capable de fonctionner.

7. Procédé de production d'un comprimé stratifié selon l'une quelconque des revendications précédentes, dans lequel le film C) se compose de ou contient:
- un, plusieurs ou tous les composés choisis dans le groupe constitué par les hydroxypropylméthylcelluloses ou l'hypromellose, le polyéthylèneglycol, le talc, les celluloses microcristallines et les monoglycérides acétylés,
et en option
- un agent de sapidité.

8. Procédé de production d'un comprimé stratifié selon l'une quelconque des revendications précédentes, dans lequel les mélanges A) et/ou B) fourni(s) à l'étape I) présentent, indépendamment les uns des autres, une humidité se situant dans la plage allant de 0,1 à 10 %, de préférence dans la plage allant de 0,5 à 5 %, de manière particulièrement préférée dans la plage allant de 1 à 2 %, par rapport au poids total du mélange A) ou B) respectif.

9. Procédé de production d'un comprimé stratifié selon l'une quelconque des revendications précédentes, dans lequel, dans la granulation en lit fluidisé en tant que procédé de granulation humide, si elle est utilisée, les constituants dudit un, des plusieurs ou de tous les composants B-ii) sont appliqués sur les constituants dudit un ou bien des plusieurs composants B-i) qui, en option, est/sont déjà revêtu(s), et un, plusieurs ou tous les paramètres suivants sont utilisés:
- température d'air amené comprise entre 20 et 80 °C, de préférence entre 25 et 60 °C, de manière particulièrement préférée entre 30 et 40 °C,
- pression d'air de pulvérisation comprise entre 1 et 7 bar(s), de préférence entre 1 et 4 bar(s), de manière particulièrement préférée entre 1,25 et 1,75 bars,
- temps de vibration comprise entre 5 et 25 s, de préférence entre 6 et 19 s, de manière particulièrement préférée entre 7 à 17 s.

10. Comprimé stratifié obtenu ou apte à être obtenu par un procédé selon l'une quelconque des revendications précédentes.

11. Comprimé stratifié selon la revendication 10, dans lequel le film C), s'il est présent, recouvre le comprimé stratifié dans une large mesure complètement, de préférence complètement, de manière particulièrement préférée complètement et uniformément.

12. Comprimé stratifié selon l'une quelconque des revendications 10 ou 11, dans lequel le comprimé stratifié est exempt d'un, de plusieurs ou de tous les ingrédients choisis dans le groupe constitué par le lactose, l'iode, le gluten, les arômes et les édulcorants, de préférence est exempt de lactose, d'iode et de gluten.

13. Comprimé stratifié selon l'une quelconque des revendications 10 à 12, pour l'utilisation en tant que médicament, de préférence pour l'utilisation dans le traitement et/ou la prévention d'une maladie métabolique et/ou d'une maladie par carence minérale, dans lequel la maladie métabolique et/ou la maladie par carence minérale est choisie de préférence dans le groupe constitué par l'hypomagnésémie, les troubles du rythme cardiaque, la migraine et l'hypertension.

14. Comprimé stratifié pour l'utilisation selon la revendication 13, dans lequel au moins un effet secondaire indésirable en raison de l'absorption accrue de magnésium dans le corps humain, due au comprimé stratifié, est réduit en ce qui concerne l'intensité dudit au moins un effet secondaire et/ou la fréquence dudit au moins un effet secondaire ou ne se produit pas, dans lequel ledit au moins un effet secondaire est ou bien sont choisi(s) dans le groupe constitué par l'effet laxatif, les selles molles, la diarrhée et les symptômes de fatigue.
